# DEMANDE DE BREVET EUROPEEN

(11) **EP 2 308 426 A1**
(43) Date de publication de la demande: **13.04.2011**
(21) Numéro de dépôt: 10184617.8
(22) Date de dépôt: 24.04.1998
(51) Int. Cl.: A61F 2/44, A61B 17/86

(54) **Implants intersomatiques en deux parties**

(30) Priorité: 25.04.1997 FR 9705137; 12.11.1997 FR 9714150
(62) Demande divisionnaire de: 06127377.7
(71) Demandeur: Stryker France, 69330 Meyzieu (FR)
(72) Inventeur: Crozet, Yves, Mahwah, NJ 07430 (US)
(74) Mandataire: Texier, Christian

(57) **Abrégé**

L'invention concerne un implant pour la chirurgie du rachis comprenant : un corps creux (10) ayant une extrémité proximale, une extrémité distale, et une paire de parois latérales (102, 103) s'étendant de ladite extrémité proximale à ladite extrémité distale, lesdites parois latérales définissant un espace intérieur généralement cylindrique ; et
un organe d'ancrage (20) apte à être inséré entre lesdites parois latérales et à l'intérieur dudit espace intérieur dudit corps creux, ledit organe d'ancrage ayant des saillies d'ancrage (22) définissant un diamètre extérieur dudit organe d'ancrage, dans lequel le diamètre extérieur des saillis d'ancrage (22) est sensiblement égal au diamètre intérieur du corps (10) dudit organe d'ancrage

## Description

La présente invention a trait d'une façon générale aux implants intersomatiques utilisables dans le traitement chirurgical du rachis.

On connaît déjà de très nombreux implants intersomatiques.

On connaît en particulier des implants de structure plus ou moins complexe, réalisées en plusieurs parties notamment pour leur conférer certaines caractéristiques de déformabilité. Ces implants connus sont désavantageux en ce qu'ils sont plus coûteux et difficiles à fabriquer, et que leur poses' avère plus délicate. Ils peuvent souffrir également d'un problème de fiabilité à terme.

On connaît également des implants qui, afin notamment de pallier tout ou partie des inconvénients ci-dessus, présentant la forme de corps creux monobloc, ou cages, munis d'aspérités sur leurs faces supérieure et postérieure pour assurer un bon blocage initial par rapport aux plateaux vertébraux sus-jacent et sous-jacent, leur caractère creux permettant une croissance osseuse à travers eux et, à terme, leur blocage définitif.

Le document FR-A-2 703 580 décrit un exemple d'un tel implant.

Ces implants monobloc connus, malgré la présence d'aspérités qui viennent s'ancrer dans les plateaux vertébraux lorsque la distraction intervertébrale nécessaire à leur mise en place est supprimée, peuvent présenter dans certains cas une stabilité insuffisante, la qualité de l'ancrage, qui s'effectue par un simple mouvement de translation, étant tributaire notamment de la dureté des os.

On connaît également par le document DE29612269 un implant qui comporte un corps extérieur dans lequel peut être engagé par vissage un élément intérieur de renforcement d'ancrage, dont les filets débordent au-dessus et au-dessous des faces supérieure et inférieure de ce corps extérieur.

La présente invention vise à améliorer ce type d'implant connu.

### RESUME DE L'INVENTION

La présente invention concerne un implant pour la chirurgie du rachis comprenant :
un corps creux ayant une extrémité proximale, une extrémité distale, et une paire parois latérales s'étendant de ladite extrémité proximale à ladite extrémité distale, lesdites parois latérales définissant un espace intérieur généralement cylindrique; et
un organe d'ancrage apte à être inséré entre lesdites parois latérales et à l'intérieur dudit espace intérieur dudit corps creux, ledit organe d'ancrage ayant des saillies d'ancrage définissant un diamètre extérieur dudit organe d'ancrage, dans lequel le diamètre extérieur des saillies d'ancrage est sensiblement égal au diamètre intérieur du corps dudit organe d'ancrage.

Des aspects préférés mais non limitatifs de l'implant selon l'invention sont les suivants :
- ledit corps creux possède une paroi d'extrémité distale reliant lesdites parois latérales à leur extrémité distale,
- la paroi distale possède un alésage taraudé,
- ladite paroi distale est arrondie pour faciliter l'insertion dudit corps creux dans ledit espace intervertébral,
- le corps creux présente la forme générale d'un U avec une extrémité distale fermée et une extrémité proximale ouverte,
- une ouverture généralement circulaire est formée à l'extrémité proximale du corps,
- au moins l'une des parois latérales présente une partie rentrante à l'extrémité proximale du corps creux,
- ledit corps creux comporte des faces supérieure et inférieure s'étendant entre lesdites extrémités proximale et distale dudit corps creux,
- lesdites faces supérieure et inférieure sont inclinées l'une par rapport à l'autre entre lesdites extrémités proximale et distale du corps creux,
- les faces supérieure et inférieure possèdent des saillies d'ancrage osseux,
- ledit corps creux les saillis d'ancrage osseux du corps creux s'étendent le long des parois de celui-ci,
- lesdites parois latérales sont partiellement cylindriques,
- les parois latérales s'étendent sensiblement sur un arc de cercle,
- au moins une des parois latérales possède une ouverture traversante,
- lesdites saillies d'ancrage dudit organe d'ancrage comprennent un filetage de vis,
- ladite extrémité distale dudit corps creux possède une ouverture circulaire délimitée par un filet rentrant prévu à au moins l'une des extrémités libres proximales des deux branches,
- ledit organe d'ancrage possède une âme creuse, à la surface extérieure de laquelle sont disposées les saillies d'ancrage,
- l'implant comprend en outre une matière promotrice de croissance osseuse disposée à l'intérieur dudit corps creux.

L'invention concerne également un implant pour la chirurgie du rachis comprend un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral. L'implant comprend en outre un organe de renforcement d'ancrage possédant sur sa surface extérieure des saillies d'ancrage osseux inscrites dans un diamètre supérieur à la hauteur hors tout du corps, ledit organe étant apte à être entraîné en rotation dans l'espace intérieur du corps.

Pour s'opposer au mouvement inverse dudit corps hors dudit espace intervertébral, et donc améliorer encore le maintien de l'implant en position, ledit corps creux possède des surfaces supérieure et inférieure pourvues de dents à bords vifs aptes à être ancrées dans lesdites vertèbres.

De préférence, lesdites dents présentent une section transversal triangulaire.

Selon un autre aspect, pour améliorer la compacité de l'implant et faciliter sa mise en place, lesdites parois latérales du corps creux sont partiellement cylindriques et coaxiales avec un axe dudit organe de renforcement d'ancrage.

Selon un autre aspect encore, pour favoriser la croissance osseuse à l'extérieur de l'implant, en particulier latéralement, lesdites parois latérales possèdent des ouvertures traversantes autorisant une croissance osseuse à travers elles.

Avantageusement, ces ouvertures comprennent des fentes allongées s'étendant essentiellement parallèlement à la direction d'insertion dudit organe dans ledit corps creux.

Pour donner au corps une plus grande largeur, on peut utiliser pour les parois latérales du corps des parois épaisses dans lesquelles sont formées des secondes ouvertures traversantes s'étendant entre les faces supérieure et inférieure du corps. Une croissance osseuse peut également être provoquée entre les deux plateaux vertébraux à travers ces secondes ouvertures traversantes.

Préférentiellement, lesdites premières ouvertures traversantes mettent en communication ledit espace intérieur avec lesdites secondes ouvertures traversantes.

On peut prévoir en outre des ouvertures traversantes mettant en communication lesdites secondes ouvertures traversantes avec les côtés extérieurs du corps.

Selon un autre aspect de l'invention, le corps creux possède une paroi d'extrémité distale reliant lesdites parois latérales, et cette paroi d'extrémité distale est arrondie pour faciliter l'insertion dudit corps creux dans ledit espace intervertébral.

L'invention propose également un implant tel que défini plus haut, dans lequel le corps creux possède une paroi d'extrémité distale reliant lesdites parois latérales, et cette paroi d'extrémité distale possède un trou taraudé pour la fixation temporaire dudit corps creux à un instrument d'insertion du corps, pour ainsi faciliter sa mise en place par le chirurgien.

Selon un autre aspect, lesdites saillies d'ancrage osseux comprennent un filetage de vis autotaraudeur.

Ce filetage peut présenter une section radiale généralement quadrangulaire.

Préférentiellement, ce filetage de vis présente une section radiale qui passe progressivement d'une section radiale essentiellement triangulaire à ladite section radiale généralement quadrangulaire à partir de l'extrémité distale dudit filetage, tandis que le diamètre du filetage de vis augmente progressivement à partir de son extrémité distale jusqu'à une partie de diamètre essentiellement constant.

On propose également selon l'invention un implant dans lequel lesdites saillies d'ancrage osseux comprennent un filetage de vis sous la forme d'une bande en hélice encerclant un espace intérieur dudit organe de renforcement d'ancrage.

Cette bande en hélice est avantageusement reliée à une fourche s'étendant à l'intérieur de ladite bande dans une direction axiale dudit organe, et cette fourche comprend de préférence deux branches s'étendant à partir d'une paroi d'extrémité proximale dudit organe de renforcement d'ancrage.

La fourche peut comprendre également des branches présentant une surface extérieure en forme de partie de tronc de cône dont le diamètre diminue de l'extrémité proximale vers l'extrémité distale de l'organe. Ceci permet de comprimer une substance promotrice de croissance osseuse préalablement placée dans l'organe de renforcement d'ancrage, lors du vissage de ce dernier.

En variante, la fourche comprend au moins deux branches possédant chacune un bord tranchant d'attaque de l'os pour ainsi accumuler des copeaux d'os à l'intérieur de l'organe et faciliter la fusion osseuse.

Il est préférable que la fourche et la bande en hélice soient réalisées d'un seul tenant.

L'invention propose également un implant dans lequel les saillies d'ancrage osseux comprennent un filetage de vis, et dans lequel ledit organe possède des moyens de blocage de celui-ci contre une rotation inverse. Préférentiellement, ces moyens de blocage comprennent une partie déformée dudit filetage dans la région de son extrémité proximale. On améliore ainsi la tenue de l'implant jusqu'à ce que la fusion soit réalisée.

Selon un autre aspect, les saillies d'ancrage osseux comprennent un filetage de vis possédant un diamètre extérieur qui diminue dans sa région distale vers son extrémité distale, pour faciliter la pénétration de ce filetage dans les plateaux vertébraux.

Par ailleurs on propose que ledit organe de renforcement d'ancrage possède une paroi d'extrémité proximale apte à essentiellement fermer une ouverture frontale dudit corps creux, de telle sorte qu'une substance promotrice de croissance osseuse placée à l'intérieur dudit organe soit comprimée pendant l'insertion dudit organe dans ledit corps creux.

Dans ce cas, l'organe de renforcement d'ancrage possède au moins une partie dont la surface externe appartient à un tronc de cône. En variante, l'organe de renforcement d'ancrage est sensiblement plus court que le corps et possède une pointe généralement conique dirigée vers ladite paroi d'extrémité distale du corps:
Il est avantageux dans ce cas que la paroi d'extrémité proximale de l'organe possède une ouverture taraudée pour la fixation temporaire dudit organe à un instrument d'insertion de l'organe.

Selon un autre aspect encore, l'invention propose un implant tel que défini plus haut, dans lequel l'organe de renforcement d'ancrage possède des moyens d'indexation pour fixer ledit organe à un instrument d'insertion de l'organe dans une relation angulaire donnée.

Selon une autre caractéristique, l'organe de renforcement d'ancrage possède à son extrémité proximale un bouchon rapporté, qui peut par exemple être vissé dans une ouverture frontale taraudée de l'organe de renforcement d'ancrage, ou encore engagé par encliquetage élastique dans une ouverture frontale de l'organe de renforcement d'ancrage.

Il est avantageux que ce bouchon possède un aménagement apte à coopérer avec un instrument permettant d'entraîner l'organe en rotation, et/ou des aménagements d'indexation angulaire de l'organe de renforcement d'ancrage avec un instrument de pose dudit organe.

On propose également selon l'invention que les saillies d'ancrage osseux comprennent un filetage de vis, et qu'au moins l'une des branches latérales du corps possède une partie rentrante formant filet apte à coopérer avec ledit filetage.

Cette partie rentrante peut être prévue seulement su l'une des branches et constituer alors la seule partie du corps coopérant par vissage avec le filetage de vis.

En outre, cette partie rentrante peut présenter un bord d'extrémité libre essentiellement rectiligne.

Selon un autre aspect, l'invention propose un implant dans lequel ledit organe est orienté en oblique, par exemple à environ 45°, par rapport à un plan du corps correspondant au plan sagittal.

Selon un autre aspect, il est proposé que des ouvertures traversantes soient prévues dans ledit organe entre l'intérieur et l'extérieur de celui-ci, lesdites ouvertures étant allongées dans une direction essentiellement circonférentielle de l'organe.

Selon un aspect encore différent, le corps possède une paroi d'extrémité distale, et l'organe de renforcement d'ancrage possède une partie d'extrémité distale apte à être vissée dans une ouverture de ladite paroi d'extrémité distale.

Le corps peut posséder également dans ce cas une paroi d'extrémité proximale incluant une ouverture plus large que la dimension extérieure dudit organe de renforcement d'ancrage et dans laquelle ledit organe peut être engagé librement.

L'invention propose par ailleurs un implant dans lequel le corps présente une paroi proximale, une paroi distale et deux parois latérales, lesdites parois définissant entre elles un espace intérieur plus grand que ledit organe de renforcement d'ancrage. On accroît ainsi l'espace affecté à la croissance osseuse entre les plateaux vertébraux sus- et sous-jacents.

Dans ce cas, l'organe de renforcement d'ancrage peut posséder une partie filetée pour son vissage dans la paroi proximale du corps, ou bien ledit organe de renforcement d'ancrage et la paroi distale du corps peuvent posséder des moyens filetés coopérant mutuellement pour la fixation dudit organe au corps.

Dans une telle configuration, les saillies d'ancrage osseux peuvent comprendre un filetage de vis ayant le même pas que ladite partie filetée ou les moyens filetés de fixation au corps.

La forme du corps, dans ce cas, est de préférence telle que les parois latérales et la paroi proximale du corps s'étendent essentiellement sur un même arc de cercle, et que ladite paroi distale soit essentiellement rectiligne.

Il est également avantageux que les faces supérieure et inférieure du corps possèdent des saillies d'ancrage osseux s'étendant le long de ses parois.

Il est également prévu selon l'invention un implant pourvu de moyens pour le montage à rotation de l'organe de renforcement d'ancrage dans l'espace intérieur du corps tout en empêchant un mouvement de translation relative entre ceux-ci.

Ces moyens de montage comprennent avantageusement une ouverture cylindrique formée dans une paroi d'extrémité distale dudit corps et un arbre prévu sur ledit organe et apte à être engagé par déformation élastique dans ladite ouverture.

Dans ce cas particulier, ledit organe de renforcement d'ancrage présente préférentiellement la forme d'une vis possédant deux plats diamétralement opposés, lesdites saillies d'ancrage osseux étant définies entre lesdits plats et des bords tranchants étant prévus aux transitons entre le filetage de la vis et les plats pour ainsi favoriser la fusion osseuse après la pose de l'implant.

Pour faciliter l'insertion de l'implant, la distance entre les plats opposés n'est pas supérieure à la distance entre les faces supérieure et inférieure dudit corps.

Selon un autre aspect de l'invention, on propose un implant pour la chirurgie du rachis, comprenant un corps essentiellement creux apte à être inséré dans un espace intervertébral, ledit corps possédant un ensemble de parois généralement parallèles définissant au moins deux espaces intérieurs situés côte à côte et exposés aux vertèbres sus- et sous-jacente qui définissent ledit espace intervertébral, ledit implant comprenant en outre au moins deux organes de renforcement d'ancrage possédant sur leur surface extérieure des saillies d'ancrage osseux inscrites dans un diamètre supérieur à la hauteur hors tout du corps, lesdits organes étant aptes à être entraînés en rotation dans des espaces intérieurs respectifs dudit corps.

Avantageusement, lesdits organes sont identiques.

L'invention prévoit par ailleurs que le corps creux puisse posséder différentes géométries, et en particulier
- des surfaces supérieure et inférieure qui sont inclinées l'une par rapport à l'autre, avec une distance entre elles qui diminue de l'extrémité proximale vers l'extrémité distale du corps ; et/ou
- des surfaces supérieure et inférieure qui sont inclinées l'une par rapport à l'autre, avec une distance entre elles qui diminue d'un premier côté latéral du corps vers le côté latéral opposé.

L'invention propose par ailleurs un jeu d'implants pour former un implant rachidien destiné à être inséré dans un espace intervertébral de la colonne vertébrale humaine en étant adapté à la géométrie dudit espace intervertébral.

Ce jeu d'implants comprend
- une pluralité de corps creux possédant chacun une paire de parois latérales délimitant un espace intérieur et aptes chacun à être inséré dans un espace intervertébral de telle manière que ledit espace intérieur soit exposé aux vertèbres sus- et sous-jacente qui définissent ledit espace intervertébral, chacun desdits corps ayant une taille et une forme spécifiques,
- au moins un organe de renforcement d'ancrage possédant sur sa surface extérieure des saillies d'ancrage osseux inscrites dans un diamètre supérieur à la hauteur hors tout des corps, ledit organe étant apte à être entraîné en rotation dans l'espace intérieur de l'un quelconque desdits corps,
   de telle sorte qu'un corps creux spécifique convenant à la configuration particulière d'un espace intervertébral donné puisse être choisi parmi ladite pluralité de corps creux.

Les tailles et formes spécifiques des corps peuvent résulter en particulier :
- de différents angles d'inclinaison entre leurs surfaces supérieures et inférieures,
- de différentes largeurs ; dans ce cas, les corps creux les plus larges présentent avantageusement des parois latérales dans lesquelles sont formées des ouvertures traversantes s'étendant entre les faces supérieures et inférieures desdits corps,
- de différentes hauteurs,
- de différentes longueurs.

Préférentiellement, un groupe déterminé de corps creux de ladite pluralité est apte à recevoir un même type d'organe de renforcement d'ancrage,

L'invention propose enfin un procédé pour positionner dans un espace intervertébral d'une colonne vertébrale humaine un implant comprenant un corps essentiellement creux possédant une paire de parois latérales entourant un espace intérieur et un organe de renforcement d'ancrage possédant sur sa surface extérieure des saillies d'ancrage osseux inscrites dans un diamètre supérieur à la hauteur hors tout du corps et apte à être entraîné en rotation dans l'espace intérieur du corps, ledit procédé comprenant les étapes suivantes :
- sélectionner à partir d'un jeu de corps creux possédant différentes formes et dimensions un corps creux adapté à la configuration dudit espace intervertébral
- remplir ledit corps creux sélectionné avec une substance promotrice de croissance osseuse
- pousser ledit corps creux dans ledit espace intervertébral de telle manière que l'espace intérieur de celui-ci soit exposé aux vertèbres sus- et sous-jacente qui définissent ledit espace intervertébral ; et
- insérer ledit organe de renforcement d'ancrage dans ledit corps creux de telle manière que lesdites saillies d'ancrage osseux s'ancrent dans lesdites vertèbres sus et sous-jacente.

Dans le cas où l'on souhaite positionner un implant dont le corps présente des parois latérales pourvues d'ouvertures traversantes s'étendant entre les faces supérieure et inférieure dudit corps, le procédé peut comprendre en outre, avant l'étape de poussée dudit corps creux dans ledit espacé intervertébral, une étape de remplissage desdites ouvertures traversantes avec une substance promotrice de croissance osseuse.

### BREVE DESCRIPTION DES DESSINS

D'autres aspects, buts et avantages de la présente invention apparaîtront mieux à la lecture de la description détaillée suivante de formes de réalisation préférées de celle-ci, donnée à titre d'exemple et faite en référence aux dessins annexés, sur lesquels
- la figure 1 est une vue en perspective d'un implant selon une première forme de réalisation de l'invention,
- la figure 2 est une vue de profil de l'implant de la figure 1 placé entre deux plateaux vertébraux,
- la figure 3 est une vue de face de l'implant de la figure 1,
- la figure 4 est une vue de dessus de l'implant des figures 1 et 3,
- la figure 5 est une vue de côté d'un élément de l'implant de la figure 1,
- la figure 6 est une vue en bout selon la flèche VI de la figure 5,
- la figure 7 est une vue en bout selon la flèche VII de la figure 5,
- la figure 8 est une vue en perspective de l'élément des figures 5 à 7,
- la figure 9 est une vue en perspective d'un implant selon une seconde forme de réalisation de l'invention,
- la figure 10 est une vue de face de l'implant de la figure 9,
- la figure 11 est une vue de dessus de l'implant des figures 9 et 10,
- la figure 12 est une vue de dessus d'un premier élément de l'implant des figures 9 à 11,
- la figure 13 est une vue de côté d'un second élément de l'implant des figures 9 à 11,
- la figure 14 est une vue en bout selon la flèche XIV de la figure 13,
- la figure 15 est une vue en bout selon la flèche XV de la figure 13,
- la figure 16 est une vue en perspective éclatée d'un implant selon une troisième forme de réalisation de l'invention,
- la figure 17 est une vue de côté de l'implant de la figure 16, assemblé,
- la figure 18 est une vue selon la flèche XIIX de la figure 17,
- la figure 19 est une vue selon la flèche XIX de la figure 17,
- la figure 20 est une vue en perspective éclatée d'un implant selon une quatrième forme de réalisation de l'invention,
- la figure 21 est une vue de côté de l'implant de la figure 20, assemblé,
- la figure 22 est une vue selon la flèche XXII de la figure 21,
- la figure 23 est une vue selon la flèche XXIII de la figure 21,
- la figure 24 est une vue en perspective éclatée d'un implant selon une cinquième forme de réalisation de l'invention,
- la figure 25 est une vue de côté de l'implant de la figure 24, assemblé,
- la figure 26 est une vue selon la flèche XXVI de la figure 25,
- la figure 27 est une vue selon la flèche XXVII de la figure 25,
- la figure 28 est une vue en perspective éclatée d'un implant selon une variante de la troisième forme de réalisation de l'invention,
- la figure 29 est une vue en perspective de l'implant de la figure 28 à l'état assemblé,
- la figure 30 est une vue de l'arrière de l'implant de la figure 29,
- la figure 31 est une vue de côté de l'implant de la figure 29,
- la figure 32 est une vue de dessus de l'implant de la figure 29,
- la figure 33 est une vue en perspective, avant assemblage, d'une variante d'exécution de la troisième forme de réalisation de l'invention,
- les figures 34 à 36 sont trois vues en perspective du corps de cette variante d'exécution,
- la figure 37 est une vue en bout, côté distal, de ce même corps,
- la figure 38 est une vue en perspective de l'organe de renforcement d'ancrage de cette variante d'exécution,
- la figure 39 est une vue en élévation de côté de ce même organe de renforcement d'ancrage,
- les figures 40 et 41 sont deux vues en perspective de l'implant selon cette même variante, à l'état assemblé,
- la figure 42 est une vue de dessus de ce même implant assemblé,
- la figure 43 est une vue en élévation de côté de ce même implant assemblé, et
- la figure 44 est une vue en bout, côté proximal, de ce même implant assemblé.

### DESCRIPTION DETAILLEE DE FORMES DE REALISATION

On notera préliminairement que, d'un figure à l'autre, des éléments ou parties identiques ou similaires sont désignés dans la mesure du possible par les mêmes signes de référence.

On notera également que les termes proximal et distal utilisés tout au long de la présente description correspondent respectivement à l'extrémité de l'implant du côté le plus proche du chirurgien au cours des opérations de pose, et à l'extrémité de l'implant la plus éloignée du chirurgien.

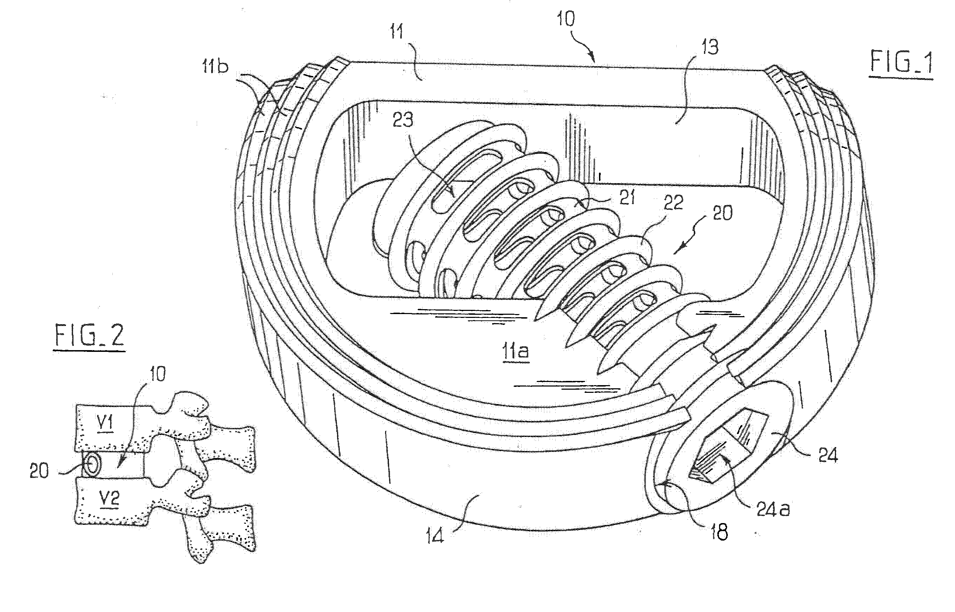

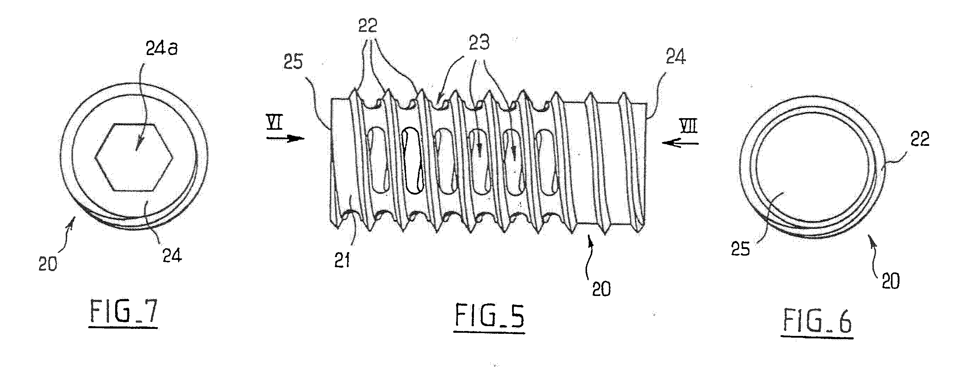

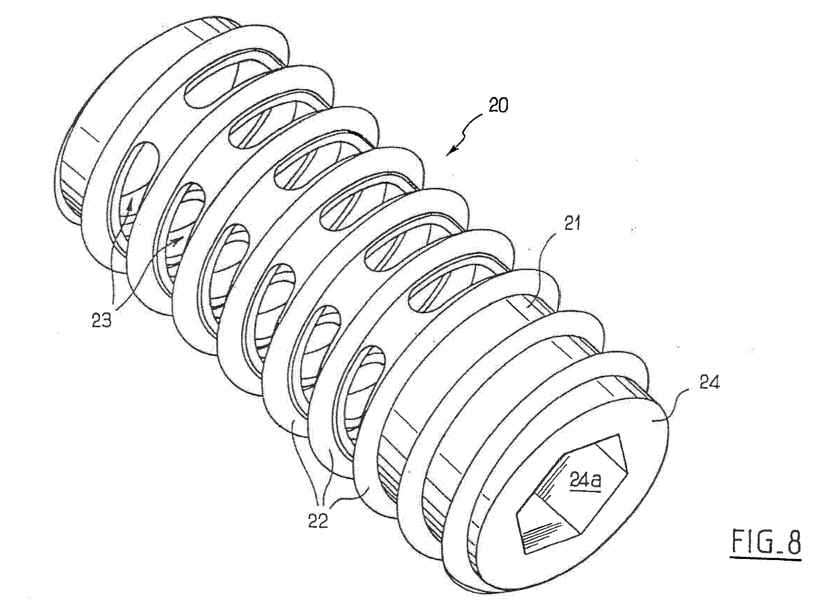

En référence tout d'abord aux figures 1 à 8, on a représenté un implant de type cage intersomatique qui est constitué de deux parties, à savoir un corps 10 et un organe de renforcement d'ancrage 20.

Le corps 10 présente la forme générale d'un anneau, avec une face supérieure 11, une face inférieure 12, une face interne 13 et une face externe 14.

Le contour du corps 10 présente une forme circulaire tronquée par une partie de contour rectiligne, sa largeur étant par exemple égale à environ quatre tiers de sa profondeur.

Le long de la partie de contour rectiligne, sur les faces supérieure et inférieure 11 et 12, sont ménagées des aspérités pour assurer un blocage de l'implant par rapport aux plateaux vertébraux sus-jacentV1 et sous-jacent V2 (voir figure 2) lorsque l'implant sera comprimé entre celles-ci.

Ces aspérités présentent en l'espèce la forme de trois nervures supérieures 11b et de trois nervures inférieures 12b, de section triangulaire et de trajectoires circulaires concentriques suivant la partie de contour circulaire du corps.

A l'opposé de la partie de contour rectiligne, le corps possède une paroi plus épaisse, matérialisée par des plages supérieure 11a et inférieure (non référencée).

Dans cette partie de paroi est ménagé un orifice traversant taraudé 18 dont l'axe s'étend en oblique, et de préférence à environ 45°, par rapport au plan vertical perpendiculaire à la paroi distale plane de l'implant, plan vertical qui correspond au plan sagittal.

En outre, l'axe de cet orifice 18, qui s'étend essentiellement horizontalement, passe sensiblement par le centre de la partie de contour circulaire, en se dirigeant vers la région opposée située à la transition entre la partie de contour circulaire et la partie de contour rectiligne.

L'implant selon l'invention comprend en outre un organe 20 destiné à conforter l'ancrage réalise au niveau des plateaux vertébraux.

Cet organe présente dans cet exemple la forme d'une âme cylindrique creuse 21 à la surface extérieure duquel est ménagé un filet hélicoïdal 22 qui est complémentaire du taraudage formé au niveau de l'orifice 18.

Entre les sections de filet adjacentes sont ménagées une pluralité d'ouvertures 23 oblongues dans la direction de l'hélice du filet, à des fins expliquées plus loin.

A son extrémité distale, l'organe 20 est fermé par une paroi pleine 25. Au voisinage de sa face opposée définissant son extrémité proximale, il comporte une partie pleine 24 dans laquelle est formée une empreinte en creux 24a, par exemple de section hexagonale, pour l'introduction d'un outil de vissage (non représenté).

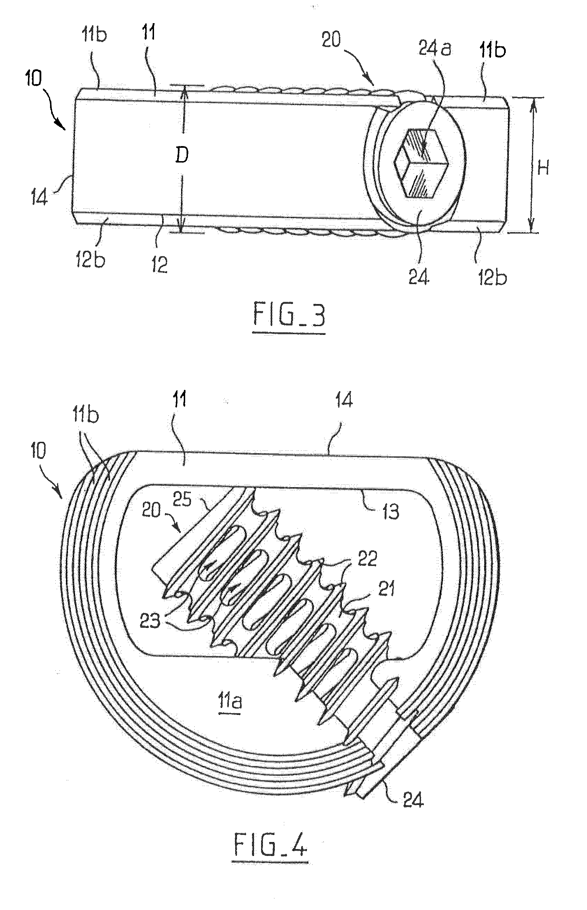

Il est important de noter, comme le montre en particulier la figure 3, que le diamètre hors tout D dans lequel s inscrit le filet 22 de l'organe 20 est légèrement supérieur à la hauteur hors tout H dans laquelle s inscrit le corps 10, ceci à des fins expliquées plus loin.

On observera ici que la longueur de l'organe 20 est telle qu'il peut être vissé dans l'orifice 18 du corps 10 jusqu' ce que la face extérieure de sa partie pleine proximale 24 vienne sensiblement dans le prolongement de la face extérieure 14 du corps 10 avoisinant ledit orifice.

On va maintenant expliquer l'utilisation d'un implant tel que décrit ci-dessus. On observera que le même type de procédure, avec les adaptations nécessaires, sera pratiquée pour les autres formes de réalisation, décrites plus loin, des implants selon l'invention.

Une distraction étant préalablement opérée entre deux vertèbres à traiter, par des moyens bien connus en eux-mêmes, le disque intervertébral est enlevé au moins partiellement et le corps 10 de l'implant, dépourvu de son organe 20, est mis en place, par voie antérieure ou postérieure. Avantageusement, l'espace intérieur du corps est préalablement rempli de greffons osseux, afin d'assurer à terme une fusion intervertébrale par ostéogenèse.

On observera ici que le contour du corps 10, avec le pan coupé en partie proximale, est tel qu'il s'inscrit bien dans la surface d'un plateau vertébral. Si nécessaire, on peut proposer au chirurgien différentes tailles de corps 10, à choisir en fonction de la morphologie rachidienne du patient, comme on le verra en détail plus loin.

Les deux vertèbres sont alors libérées, et un premier blocage du corps entre les plateaux vertébraux V1 et V2 est assuré à l'aide des nervures 11b, 12b.

L'organe 20 est alors vissé à l'aide d'un outil dans l'orifice 18. Au cours de ce mouvement, la partie sommitale du filet 22, qui déborde légèrement vers le haut et vers le bas par rapport aux parties sommitales des nervuresl11b, 12b, entaille les faces en vis-à-vis des plateaux vertébraux sus-jacent et sous-jacent à la manière d'une vis auto-taraudeuse, et réalise ainsi un ancrage supplémentaire qui vient fermement bloquer l'implant par rapport à ces plateaux.

En outre, la rotation de l'organe 20 à mesure qu'il pénètre dans l'espace intérieur, rempli de greffons osseux, du corps 10, fait qu'une partie de ces greffons va migrer à travers les ouvertures 23 jusque dans l'espace intérieur de l'organe creux 20. La croissance osseuse va donc également se produire à travers l'organe 20, ce qui va avantageusement bloquer l'organe 20 vis à vis de toute rotation notamment inverse risquant d'affecter à terme la tenue de l'implant. En variante, on peut prévoir également que l'organe 20 soit préalablement rempli de greffons osseux.

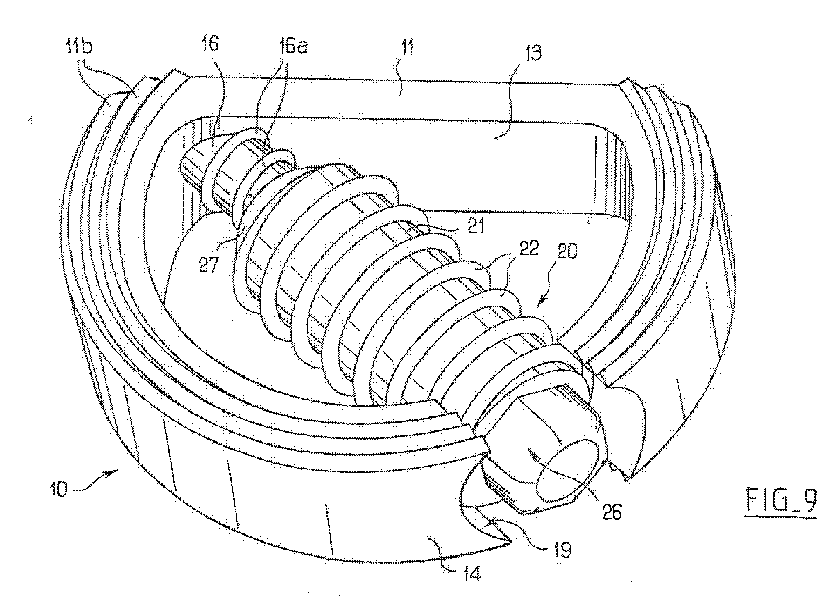

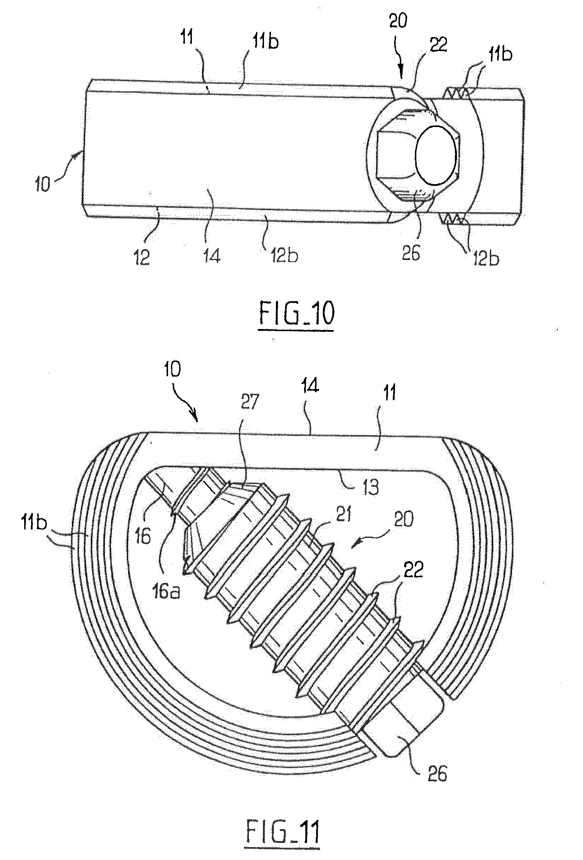

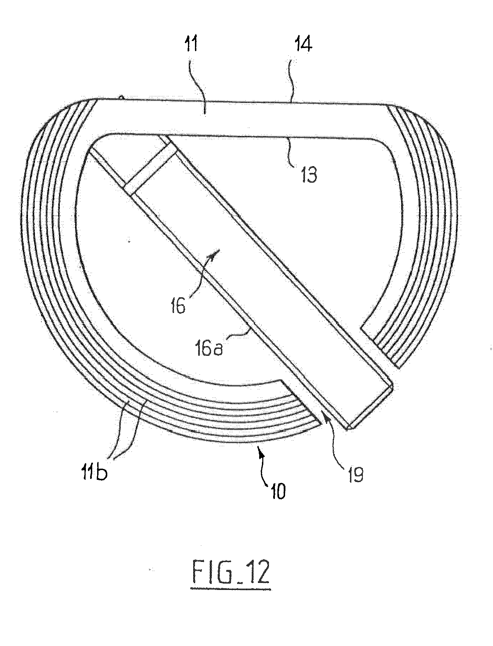

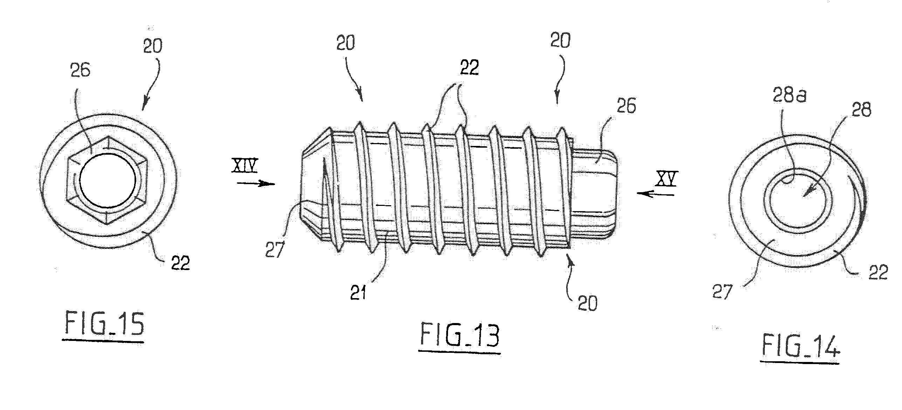

Les figures 9 à 15 illustrent une seconde forme de réalisation de la présente invention.

Sur ces figures, des éléments ou parties identiques ou similaires à ceux des figures 1 à 8 sont désignés par les mêmes signes de référence, et l'on écrira seulement les différences de cette seconde forme de réalisation par rapport à la première.

On notera tout d'abord que le corps 10, qui présente le même contour que dans le cas des figures 1 à 8, présente ici une paroi d'épaisseur essentiellement constante sur toute sa périphérie.

Ce corps comporte, en lieu et place de l'orifice taraudé 18 de la première forme de réalisation, un orifice traversant lisse 19.

Par ailleurs une tige cylindrique 16 pourvue d'un filetage 16a s'étend dans l'axe de l'orifice 19 à partir de la région opposée du corps 10, située essentiellement à la transition entre ses parties de contour circulaire et de contour droit.

En outre, l'organe 20, qui présente sensiblement le, même contour extérieur que dans le cas de la première forme de réalisation, est plein, mis à part un alésage central 28 débouchant au niveau de sa face postérieure et dans lequel est formé un taraudage 28a complémentaire du filetage 16a formé sur la tige saillante 16 du corps.

On observera ici que le pas d'hélice du filet 16a et du taraudage associé 28a est choisi sensiblement ou exactement au pas d'hélice du filet 22, qui reste présent à la surface extérieure de l'organe 20.

Autour du débouché de l'alésage 28 est prévue une partie tronconique 27.

On observera enfin que l'organe 20 possède sur sa face antérieure un aménagement de vissage qui est dans ce cas constitué par une tête saillante 26 de section par exemple hexagonale.

L'implant selon cette seconde forme de réalisation s'utilise essentiellement de la même manière que celle précédemment expliquée.

La différence essentielle réside en ce que l'organe 20 est vissé sur la tige 16 à la manière d'un écrou, la taille de l'orifice 19 étant choisie de manière à ne pas faire obstacle à ce vissage.I1 suffit à cet égard de choisir un diamètre d'orifice 19 légèrement supérieur au diamètre hors tout du filet 22. On notera que la partie tronconique 27 de l'organe 20 permet de faciliter l'introduction de l'extrémité postérieure dudit organe dans l'orifice 19 préalablement au vissage.

Le pas du filet 16a étant le même que celui du filet 22,la progression de l'organe 20 dans le corps 10 à mesure que l'organe est entraîné en rotation est telle que le filet 22 vient ici encore mordre dans les plateaux vertébraux à la manière d'une vis auto-taraudeuse.

En particulier, on peut prévoir que les faces annulaires supérieure et inférieure 11 et 12 du corps 10 s'étendent dans des plans présentant une légère obliquité l'un par rapport à l'autre, de manière à s'adapter à la forme de l'espace intervertébrale concerné. Ainsi l'on peut proposer au chirurgien, comme on le verra plus loin des corps 10 présentant des obliquités différentes pour s'adapter à la morphologie des vertèbres à traiter.

Dans ce cas, la forme de réalisation des figures 9 à 15 est avantageuse en ce que l'on peut donner au contour extérieur de l'organe 20 une forme légèrement tronconique, de telle sorte que le degré de débordement du filet 22 par rapport aux sommets des nervures 11a et 11b reste essentiellement constant de l'avant vers l'arrière de l'implant et que, les faces concernées des vertèbres étant sensiblement parallèles aux faces supérieure et inférieure du corps 10, l'ancrage par le filet soit essentiellement de même importance de l'extrémité proximale jusqu'à l'extrémité distale.

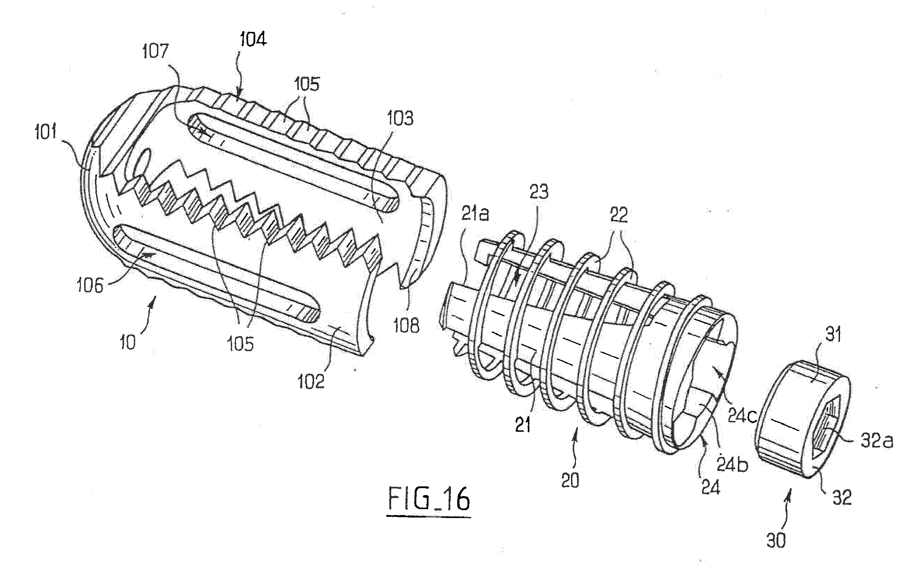

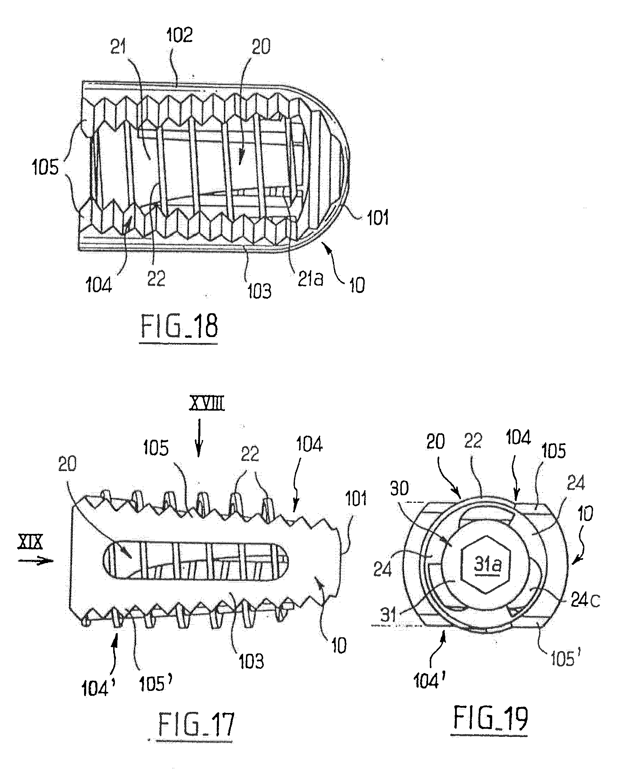

En référence maintenant aux figures 16 à 19, on a représenté un implant selon une troisième forme de réalisation de l'invention, qui comprend un corps 10 présentant en section horizontale la forme générale d'un U, avec un fond 101 ou paroi distale et deux parois ou branches latérales essentiellement parallèles 102, 103. Ce corps comporte des faces supérieure et inférieure en forme de U, respectivement 104, 104', sur lesquelles sont ménagées des dents d'ancrage osseux, respectivement 105, 105', en l'espèce des dents à bords vifs de profil triangulaire, qui remplissent un rôle analogue à celui des nervures 11b des formes de réalisation précédentes. On observe en particulier sur la figure 17 que les faces supérieure et inférieure 104, 104' vont en se rapprochant légèrement l'une de l'autre en direction de la région de fond 101,

Dans la paroi distale 101 est formé un alésage taraudé 1010 permettant la fixation temporaire d'un instrument, non représenté et classique en soi, pour la pose du corps dans l'espace intervertébral,

Les branches 102, 103 définissent un espace intérieur généralement cylindrique, à des fins expliquées plus loin.

Les deux branches latérales 102, 103 du corps comportent chacune une fente longitudinale traversante, respectivement 106, 107, ces fentes étant destinées à autoriser une croissance osseuse latéralement.

A l'extrémité ouverte du corps 10, a l'opposé de son fond 101, est formée une ouverture généralement circulaire délimitée par un filet rentrant 108 prévu aux extrémités libres proximales des deux branches 102, 103.

L'implant Comporte un organe de renforcement d'ancrage 20 pourvu d'une âme creuse dont la surface externe est légèrement tronconique, en se rétrécissant de son extrémité proximale vers son extrémité distale. A la surface extérieure de l'âme 21 est formé un filetage continu 22.

Ce filetage 22, en forme de bande plate conformée en hélice, est apte à coopérer avec le filet rentrant 108 du corps 10 pour permettre le vissage de l'organe 20 à l'intérieur dudit corps.

Comme on l'observe en particulier sur la figure 16, l'âme 21 est constituée par trois branches longitudinales angulairement décalées, séparées par des espaces vides longitudinaux 23.

Chacune de ces branches comporte un bord d'attaque (c'est-à-dire le bord avant dans la direction du vissage de l'organe 20) qui est tranchant en 21a, de manière à constituer un organe de raclage de la matière osseuse des vertèbres sus- et sous-jacente. De la sorte, le vissage de l'organe 20 va permettre de remplir l'espace Intérieur de l'implant avec des copeaux osseux, ce qui va favoriser la prise de greffe et finalement la soudure par croissance osseuse entre les deux vertèbres.

On observera ici que le diamètre extérieur du filetage 22 est de préférence très voisin du diamètre intérieur du corps 10, de manière à assurer, lors du vissage de l'organe 20, son guidage à l'intérieur du corps.

Enfin l'organe 20 comporte, dans sa partie proximale 24 formant une douille, une ouverture délimitée par une pluralité de bossages 24b séparés par des zones en creux 24c. Les bossages 24b, qui constituent l'amorce des branches 21 de l'âme, portent à leur surface intérieure un taraudage.

L'implant comporte enfin un bouchon généralement cylindrique 30 possédant à sa surface extérieure un filetage 31 apte à coopérer avec le taraudage défini par les bossages 24b. La face postérieure 32 de ce bouchon est munie d'une empreinte en creux 32a pour un outil de vissage,

L'implant tel que décrit ci-dessus s'utilise comme de la façon suivante
- le corps 10, dépourvu de l'organe 20, est mis en place entre les vertèbres à traiter;
- l'organe 20, dépourvu de son bouchon 30, est rempli de greffons osseux par son ouverture postérieure, puis le bouchon est mis en place dans cette ouverture pour éviter que les greffons ne s'échappent;
- l'organe 20 muni de son bouchon est ensuite vissé dans le corps 10 déjà en place, à l'aide d'un outil de vissage engagé dans l'empreinte 32a; au cours de cette opération, le filetage 22 de l'organe 20 vient s'ancrer dans les surfaces en vis-à-vis des vertèbres sus- et sous-jacentes, avec le cas échéant détachement de copeaux osseux; en outre, les bords tranchants 21a des trois branches 21 de l'âme de l'organe 20 attaquent les vertèbres pour détacher des copeaux qui vont compléter le remplissage de l'espace intérieur de l'organe 20; enfin la forme tronconique de l'âme 21 de l'organe 20 assure, à mesure de son avancée, une compression d'une partie de cette matière osseuse contre les parois des vertèbres, pour favoriser la greffe.

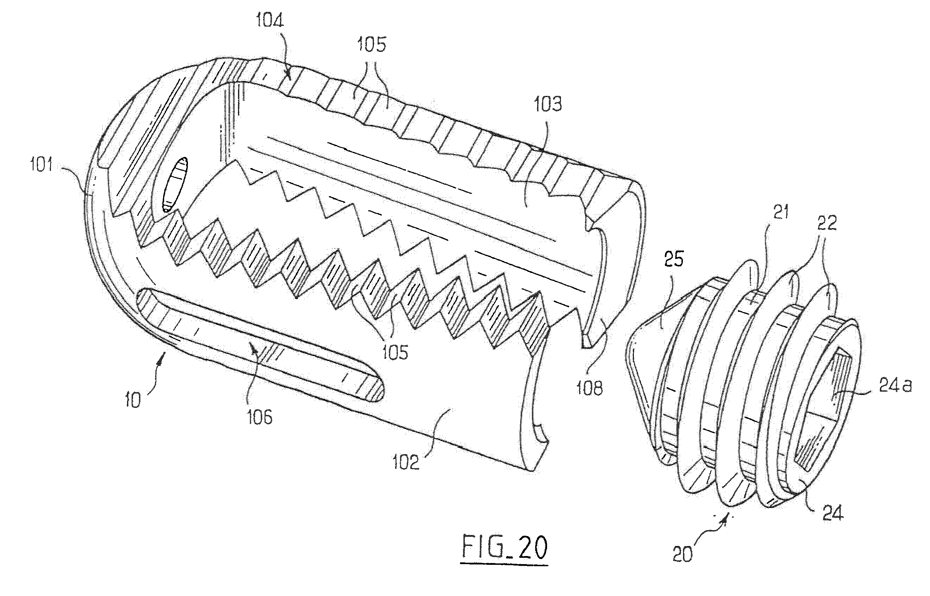

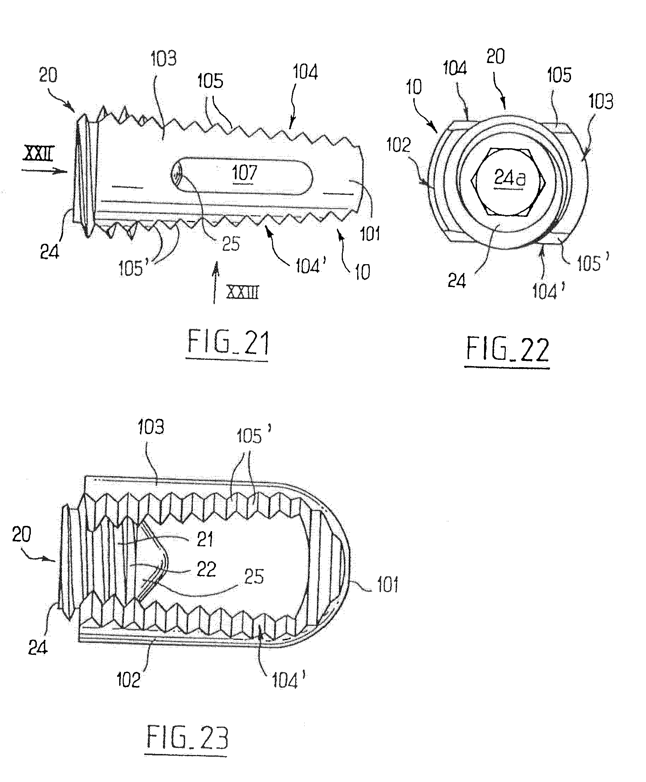

Les figures 20 à 23 illustrent une quatrième forme de réalisation de l'invention, dans laquelle le corps 10 est similaire à celui des figures 16 à 19, et ne sera pas à nouveau décrit dans son ensemble. On observera toutefois que, dans cet exemple, seule la branche 102 du corps est pourvue d'une fente traversante 106, tandis que l'autre branche en est dépourvue. Ce type de corps est utilisé avantageusement lorsque l'on utilise deux implants selon l'invention dans un même espace intervertébral, Dans ce cas, les implants sont disposés côte à côte de telle sorte que les fentes respectives des deux corps se trouvent du côté interne, ceci afin de favoriser la fusion avec des greffons osseux placés dans la région de l'espace intervertébral située entre les deux implants.

L'organe de renforcement d'ancrage 20 présente dans ce cas la forme d'un bouchon fileté sensiblement plus court que le corps 10 en direction axiale, Cet organe possède une âme pleine cylindrique 21 pourvue d'un filetage 22 apte à coopérer avec le filet 108 du corps 10, de la même manière que précédemment.

La face postérieure de l'organe 20 possède une empreinte en creux 24a pour outil de vissage, tandis que sa face antérieure 25 présente la forme d'un cône à sommet arrondi.

L'implant selon cette forme de réalisation est destiné à être utilisé en remplissant le corps 10 de greffons osseux de façon relativement dense. De la sorte, la pénétration de l'organe 20 au cours du vissage assure, outre le renforcement d'ancrage réalisé à l'aide du filetage 22, une compression des greffons osseux situés dans le corps 10, pour les solliciter en particulier en direction des plateaux vertébraux sus- et sous-jacents et améliorer la fusion.

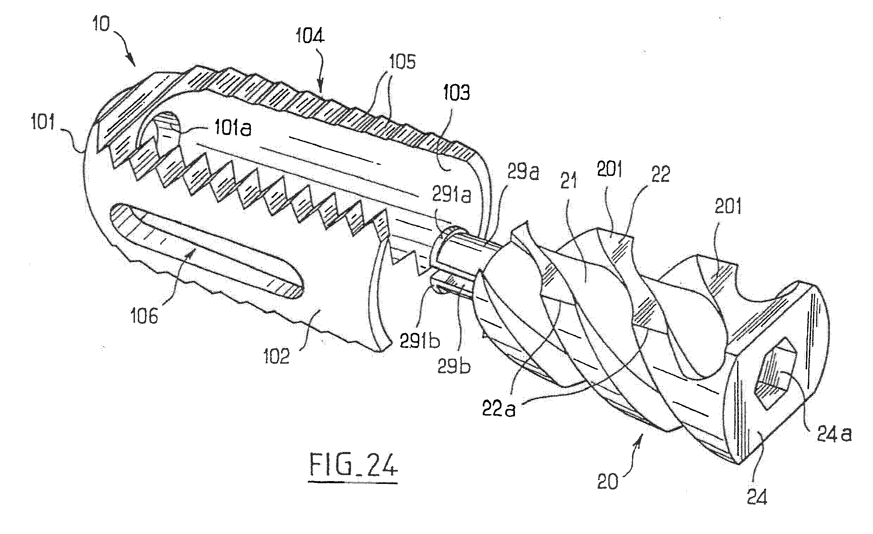

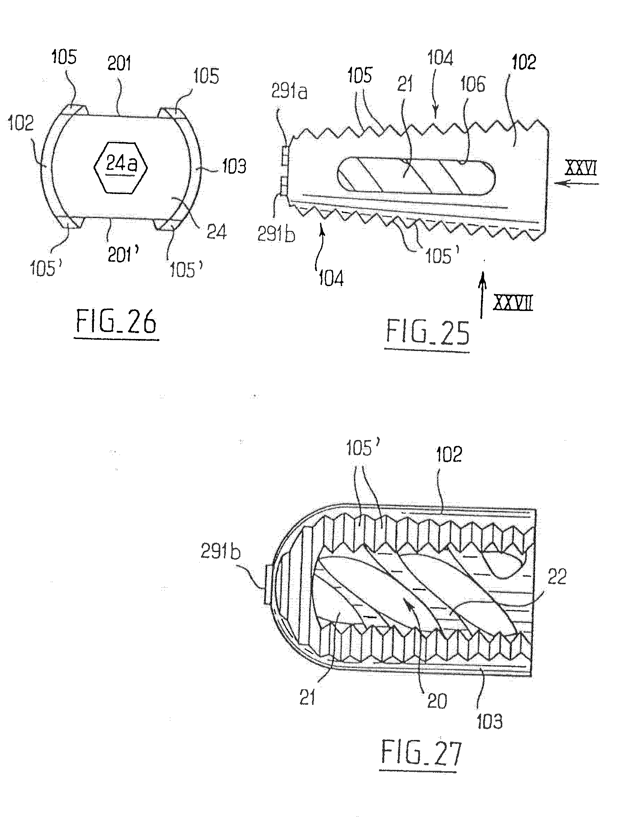

Les figures 24 à 27 illustrent une cinquième forme de réalisation de1 invention. Le corps 10 de l'Implant se distingue de celui des troisième et quatrième formes de réalisation essentiellement par le fait que la partie de fond 101 possède une ouverture cylindrique traversante 101a disposée selon l'axe du corps, et que l'ouverture d'entrée située à l'opposé du fond 101 est dépourvue du filet 108 des réalisations précédentes.

L'organe de renforcement d'ancrage 20 présente ici la forme d'une vis à filet large, qui possède à son extrémité antérieure un prolongement en forme d'arbre formé de deux pattes essentiellement semi-cylindriques axiales 29a, 29b présentant à leur extrémité libre une surépaisseur, respectivement 291a, 291b.

Ces deux pattes présentent un diamètre extérieur légèrement inférieur au diamètre de l'ouverture 101a du corps 10, et une épaisseur réduite de manière à ce que leur déformabilité élastique permette, préalablement à la pose par le chirurgien, l'encliquetage de l'organe 20 dans le corps 10, ledit organe 20 étant ainsi bloqué contre toute translation mais étant libre en rotation et guidé d'une part par l'ouverture 101a, et d'autre part par les faces internes des deux branches 102, 103 du corps 10.

Une autre particularité essentielle de cette forme de réalisation réside en ce que l'organe 20 est délimité par deux pans coupés ou plats, respectivement 201, 201', qui confèrent à l'organe, dans sa position angulaire telle qu'illustrée sur la figure 24, une épaisseur sensiblement égale à l'épaisseur du corps 10, et ceci tout le long de celui-ci.

On observe également qu'à la transition entre le filetage 22 (de section carrée) et les pans coupés 201, 201', les filets forment chacun un angle vif 22a.

Par ailleurs, comme dans certaines des formes de réalisation précédentes, la partie proximale 24 de l'organe 20 est munie d'une empreinte en creux 24a pour un outil de vissage.

La pose de l'implant par le chirurgien s'effectue comme suit
- l'implant complet,c'est-à-dire le corps 10 abritant l'organe 20 préalablement encliqueté et auquel on a donné l'orientation angulaire de la figure 24, est engagé par impaction dans l'espace intervertébral, cette opération étant facilitée par le fait que l'organe 20 ne déborde pas par rapport aux limites du corps 10;
- l'organe 20 est ensuite tourné sur lui même à l'aide d'un outil approprié engagé dans l'empreinte 24a, si bien que les bords vifs des filets 22 viennent attaquer la matière osseuse des plateaux vertébraux sus et sous-jacènts, en arrachant de ce fait des copeaux osseux qui vont remplir les espaces libres existant entre le corps 10 et l'organe 20 pour contribuer à la fusion.

Du fait que l'organe 20 est bloqué contre toute translation par rapport au corps 10, et n'a donc pas vocation à être vissé dans celui-ci ou dans les plateaux vertébraux, on donne avantageusement aux filets 22 un pas d'hélice important, de telle sorte que l'action de vissage privilégie un glissement réciproque des filets 22 par rapport aux plateaux vertébraux, sans induire d'effort axial suffisamment important pour déplacer l'implant selon cette direction.

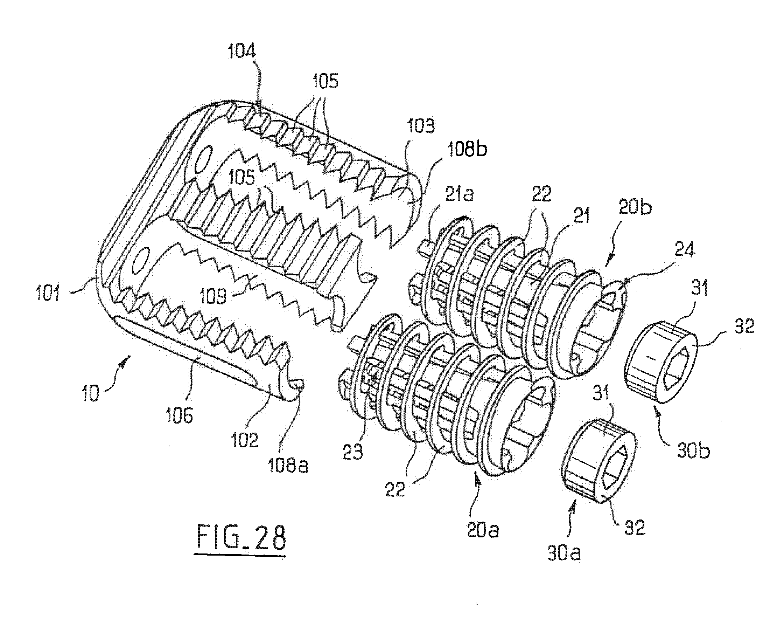

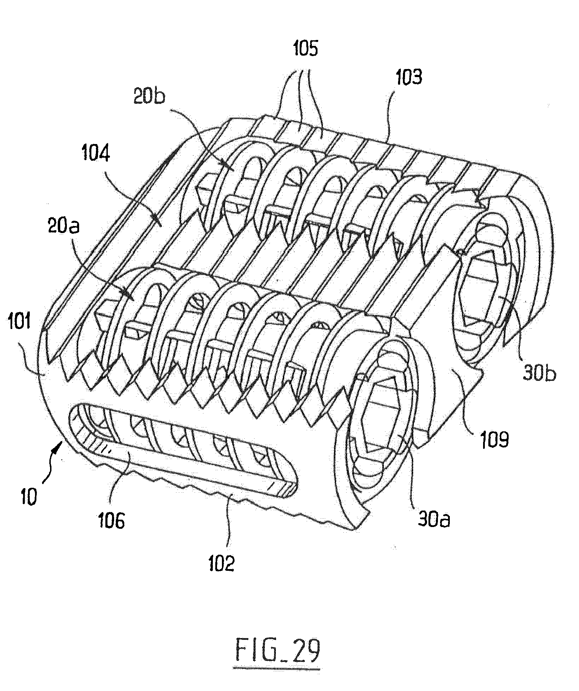

Maintenant en référence aux figures 28 à 32, on va décrire une première variante d'exécution de la troisième forme de réalisation de l'invention décrite plus haut en référence aux figures 16. à 19. Dans la description qui suit, on ne reprendra pas les éléments déjà décrits en référence aux figures 16 à 19, mais seulement les différences essentielles apportées par la variante.

Selon cette variante, le corps 10 est élargi et conçu pour recevoir deux organes de renforcement d'ancrage, respectivement 20a et 20b. A cet effet, le corps 10 est élargi et possède deux branches latérales 102 et 103 ainsi qu'une branche intermédiaire médiane 109 s'étendant entre les branches 102 et 103.

Les branches 102 et 109 définissent un premier logement pour l'organe 20a, tandis que les branches 103 et 109 définissent un second logement pour l'organe 20b, les axes de ces deux logements étant ici mutuellement parallèles mais pouvant le cas échéant adopter une certaine obliquité. Ces deux logements possèdent de référence la même configuration que le logement unique de la troisième forme de réalisation, et les organes 20a et 20b sont de préférence semblables à l'organe 20 de cette même forme de réalisation.

De même, le corps 10 est pourvu de dents d'ancrage osseux 105, 105'.

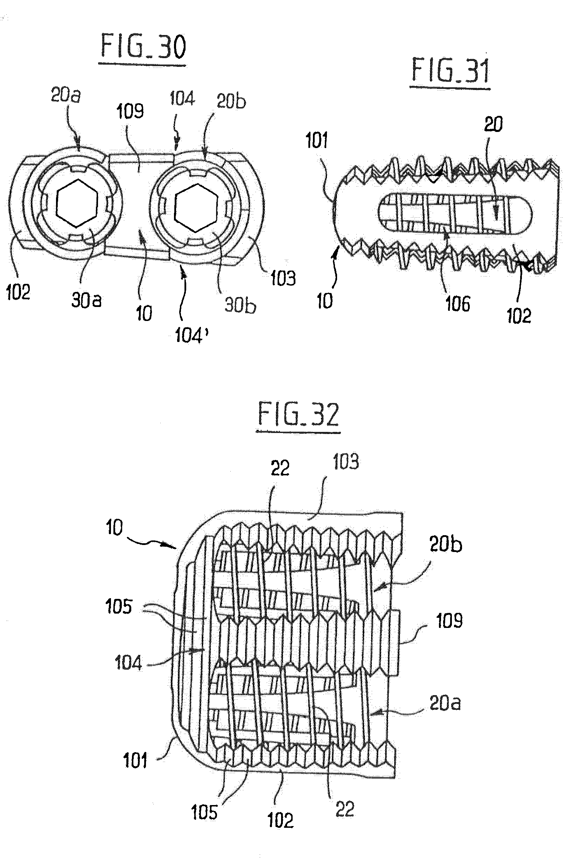

On observera ici, comme le montrent tout particulièrement les figures 30 et31, que les faces supérieure et inférieure 104 et 104' du corps 10 présentent une double obliquité, l'une correspondant à un rapprochement de ces faces en direction du fond des logements, et l'autre correspondant à un rapprochement de ces faces dans une direction latéral (de la droite vers la gauche sur la figure 30, mais le rapprochement inverse pouvant être réalisé en retournant simplement le corps 10 sur lui-même.

Cette double obliquité permet au corps 10 d'être implanté en biais en tout en rétablissant la lordose lombaire dans le plan sagittal,

Par ailleurs, la largeur accrue de l'implant permet d'assurer un appui plus stable entre les deux plateaux vertébraux, tandis que la présence de deux organes de renforcement d'ancrage 20a et 20b permet de renforcer la résistance au glissement par rapport à ces plateaux.

Bien entendu, cette variante de réalisation de l'invention peut s'appliquer à tous les autres implants décrits dans le présent mémoire, seule une adaptation du corps 10, à la portée de l'homme du métier, étant nécessaire.

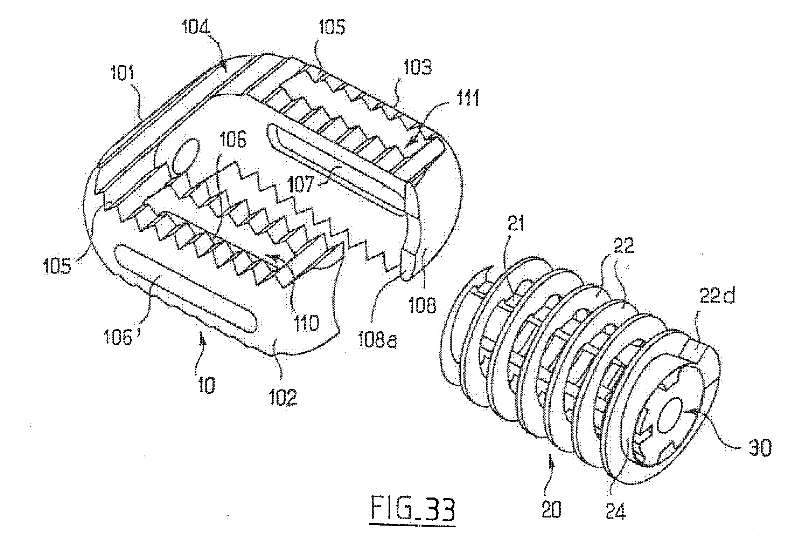

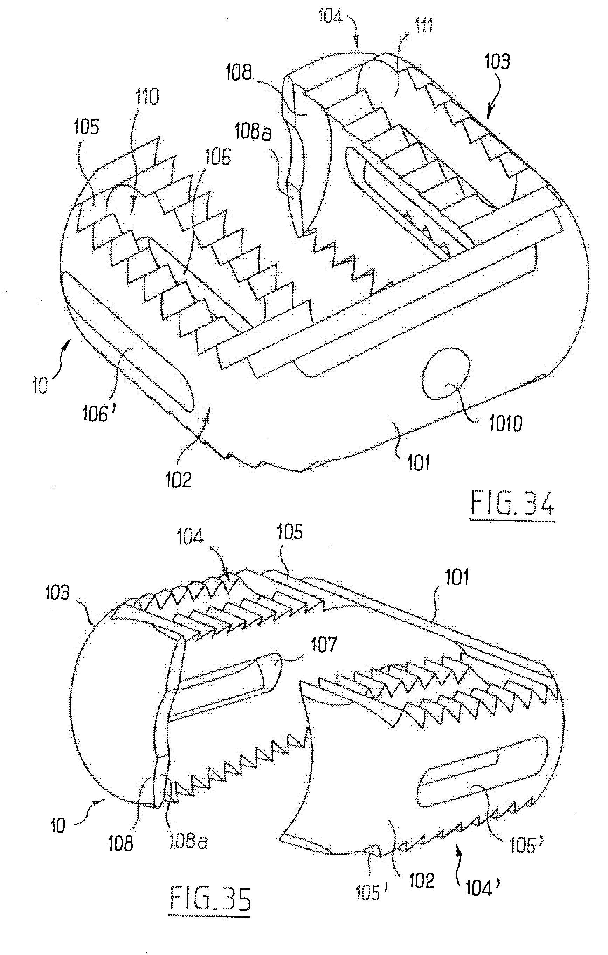

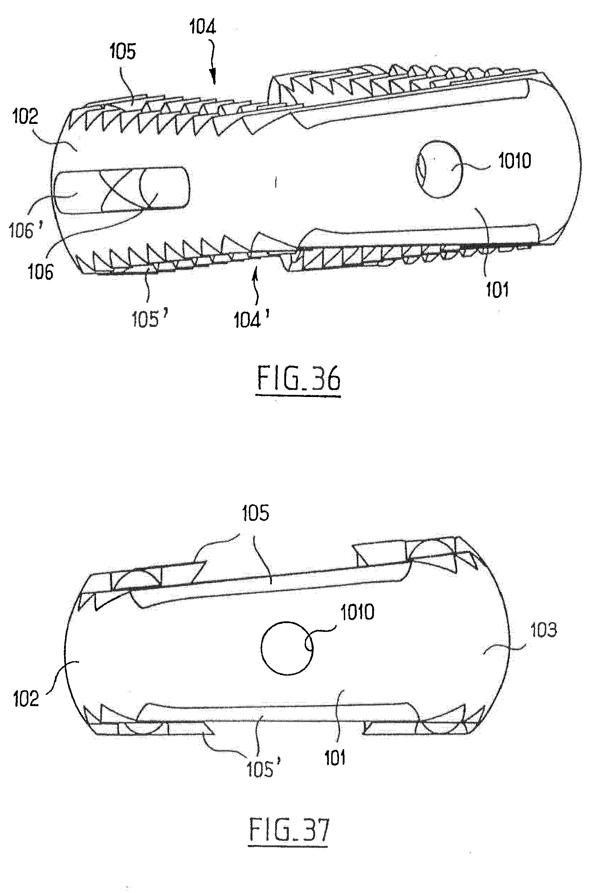

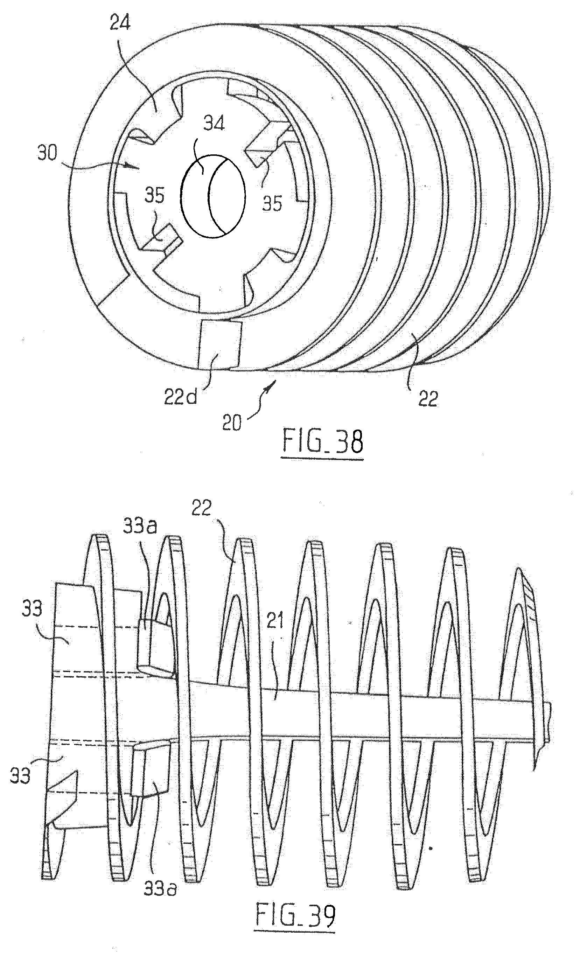

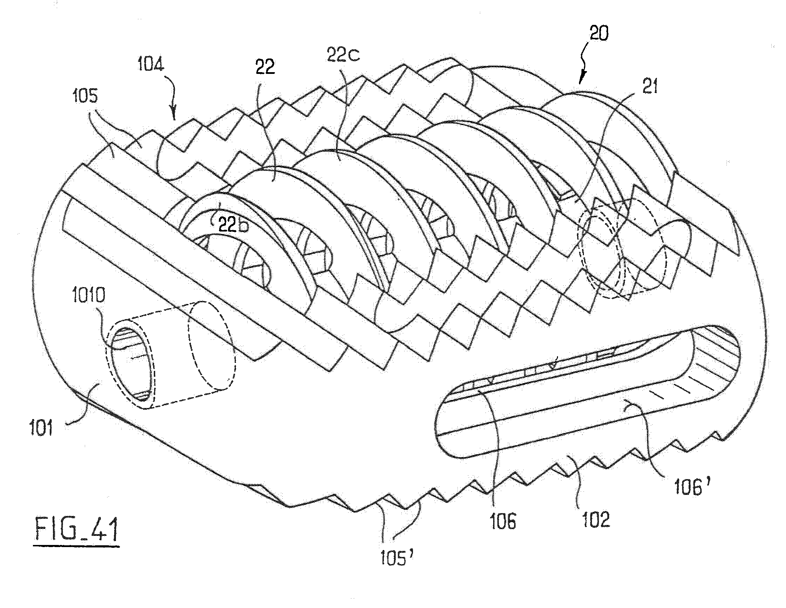

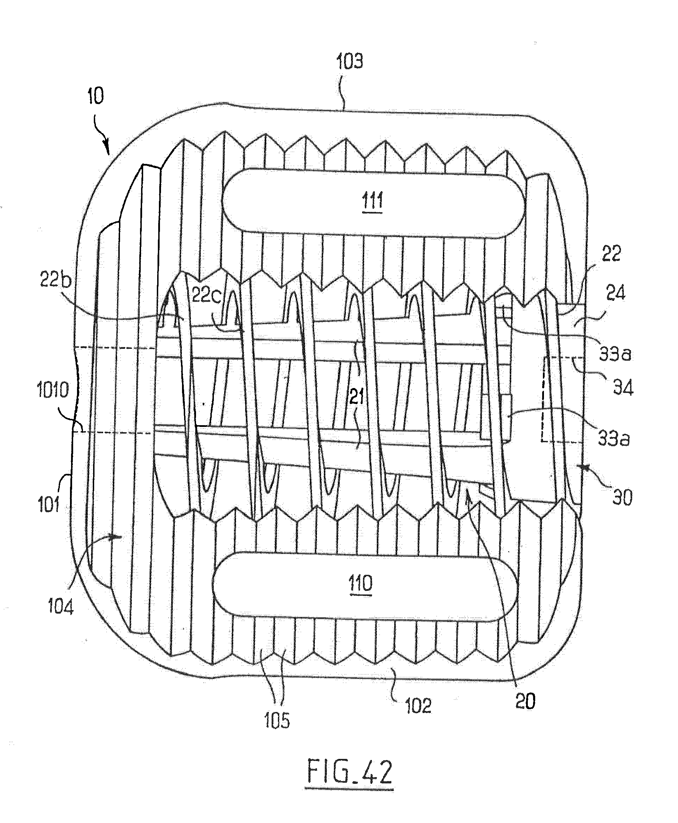

En référence maintenant aux figures 33 à 44, on va décrire une autre variante de réalisation de l'implant décrit en référence aux figures 16 à 19.

Selon cette variante, le corps extérieur 10 de l'implant comprend, de la même manière que précédemment, une forme générale de U avec deux branches latérales 102 et 103 réunies par une paroi d'extrémité distale 101, avec des transitions arrondies.

Pour accroître la largeur de l'implant, et donc améliorer sa stabilité, on prévoit que les branches latérales 102 et 103 présentent en direction latérale une épaisseur sensiblement supérieure à celle des branches 102 et 103 décrites en référence aux figures 16 à 19.

De préférence, cette épaisseur est choisie de manière à donner à la largeur hors tout de l'implant une valeur par exemple égale à environ 1,5 à 2,5 fois le diamètre de l'organe de renforcement d'ancrage 20.

En outre, pour améliorer encore la fusion osseuse entre les plateaux vertébraux sus- et sous-jacents, on prévoit des ouvertures traversantes oblongues 110 et 111 s'étendant par exemple verticalement entre la face supérieure 104 et la face inférieure 104' du corps, de telle sorte que les branches latérale 102 et 103 possèdent chacune une double paroi. Dans chacune des ces parois est par ailleurs ménagée une ouverture oblongue généralement horizontale, respectivement 106, 106' et 107, 107' qui permettent à l'espace intérieur du corps 10 de s'ouvrir latéralement sur l'extérieur du corps, en traversant les deux doubles parois et les ouvertures traversantes 110, 111 respectivement..

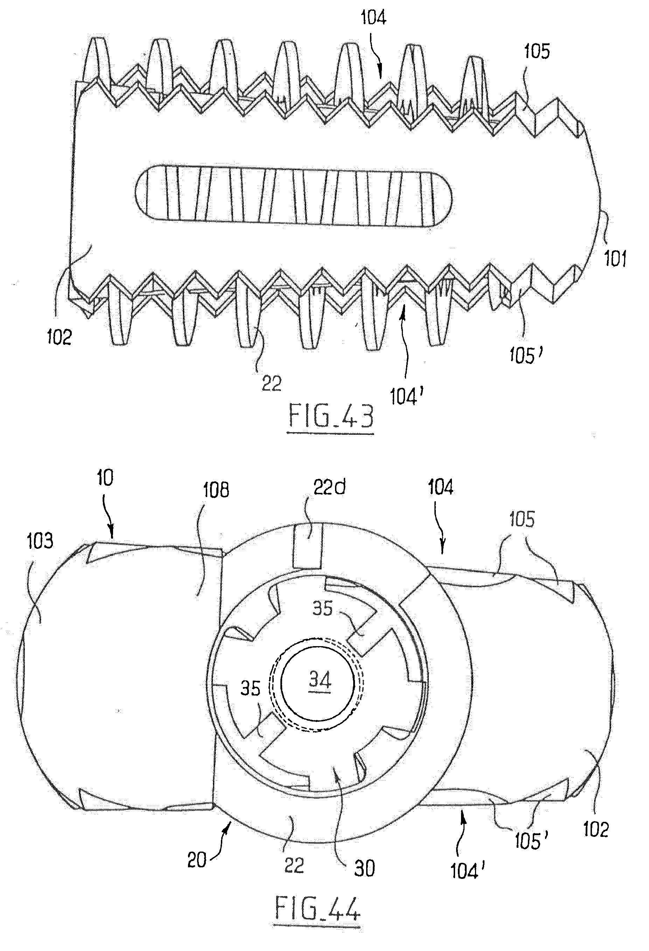

On observe par ailleurs, comme le montrent les figures 43 et 44, que les faces supérieure et inférieure 104 et 104' du corps présentent l'une par rapport à l'autre une double obliquité, d'une part en direction latérale, et d'autre part de l'extrémité proximale vers l'extrémité distale.

L'organe de renforcement d'ancrage 20 présente une construction semblable à celle qui a été décrite en référence aux figures 16 à 19. Il comprend pour l'essentiel une fourche intérieure possédant deux branches 21, desquels est solidaire une bande hélicoïdale 22 formant un filet d'ancrage osseux, les parties 21 et 22 étant de préférence réalisées d'un seul tenant.

Le filet 22 est de préférence ici un filet auto taraudeur, permettant de réaliser un vissage en attaquant directement les plateaux vertébraux sus- et sous-jacents, sans avoir à réaliser préalablement à la pose un taraudage dans ces plateaux vertébraux. A cet effet, le filet 22 présente dans sa région d'extrémité distale une section radiale 22b en forme de pointe tournée vers l'extérieure, et cette section varie progressivement, par exemple sur l'étendue d'une fraction de tour, jusqu'à une section radiale rectangulaire 22c. En outre, le diamètre du filet 22 augmente progressivement de son extrémité distale jusqu'à ladite partie de section rectangulaire qui est ici de diamètre constant.

On observe également que les faces extérieures des branches 21 présentent une forme tronconique, dont le diamètre diminue de l'extrémité proximale vers l'extrémité distale, à des fins expliquées plus loin.

Les deux branches sont réunies au niveau, d'une douille 24 qui présente la forme d'une bague cylindrique réalisée de préférence d'un seul tenant avec lesdites branches.

Dans cette douille 24 peut être monté par encliquetage élastique à partir de l'extérieur un bouchon 30 qui possède une série de pattes flexibles de verrouillage 33, en l'espèce deux paires de pattes, s'engageant dans l'ouverture centrale de la douille 24 et dont les extrémités en forme de dents 33a peuvent venir s'accrocher sur le bord interne de la douille 24.

L'organe 20 et son bouchon 30 sont solidarisés ensemble vis-à-vis de la rotation par le fait que chaque paire de pattes 33 vient entourer étroitement l'amorce d'une ranche respective 21 de la fourche.

Le bouchon 30 possède également un alésage taraudé 34 disposé centralement et permettant de recevoir l'extrémité en forme de tige filetée d'un instrument, connu en soi et non représenté, de pose de l'organe 20.

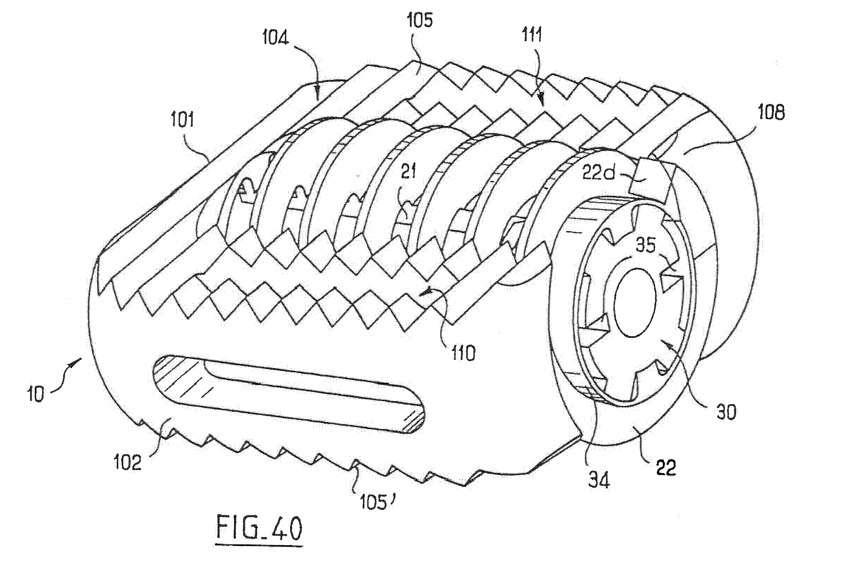

On observe par ailleurs sur les figures 40 et 44 que deux encoches diamétralement opposées 35, ménagées de part et d'autre de l'alésage taraudé 34, permettent de réaliser une indexation angulaire de l'instrument précité, alors équipé d'aménagements complémentaires, par rapport au bouchon 30 et donc à l'ensemble de l'organe 20 de renforcement d'ancrage.

On observe ensuite, en particulier sur la figure 44, que le filet 108 permettant d'assurer une coopération vissée entre le corps extérieur 10 et l'organe 20 est prévu seulement sur l'une des branches latérales 102 du corps, sous la forme d'une membrure rentrante se terminant par un bord 108a généralement rectiligne.

Enfin l'on observe, en particulier sur les figures 33, 38 et 40, que l'extrémité du filet 22 côté proximal est déformée,comme Indiqué en 22d, cette déformation étant réalisée en direction du tour de filet adjacent, c'est-à-dire vers l'extrémité distale.

Cette déformation permet de conférer au filet 22 une fonction de blocage contre la rotation inverse, et donc d'éviter que l'organe de renforcement d'ancrage 20, après la pose mais avant la fusion osseuse, ne risque de se désolidariser du corps 10.

La pose de l'implant décrit ci-dessus en référence aux figures 33 à 44 s'effectue avec les étapes successives suivantes
- tout d'abord, on remplit les ouvertures 110 et 111 du corps 10 de matière promotrice de croissance osseuse, telle que des greffons osseux
- après distraction si nécessaire, on insère ensuite le corps dans l'espèce intervertébral ;
- on remplit l'organe 20 de renforcement d'ancrage avec une matière promotrice de croissance osseuse, puis on ferme cet organe 20 à son extrémité proximale par encliquetage du bouchon 30 ;
- par vissage, on engage ledit organe dans le corps préalablement posé ; Il est à noter ici que la forme tronconique des deux branches 21 de la fourche de l'organe 20 permet, à mesure de l'avancée de l'organe 20, de comprimer la matière de croissance osseuse et donc d'assurer un bon contact d'une part avec les plateaux vertébraux sus- et sous-jacents, et d'autre part avec la matière de croissance osseuse préalablement placée dans les ouvertures 110 et 111, via les ouvertures 108 et 109.

Les implants selon l'invention sont bien évidemment réalisés en un matériau biocompatible de rigidité appropriée, tel qu'un alliage de titane ou un acier inoxydable.

De façon avantageuse, on propose au chirurgien des Implants selon l'invention sous la forme d'un jeu d'implants de formes et de dimensions différentes, qui permettent de choisir l'implant, et en particulier le corps 10, le mieux adapté à la morphologie du site de pose.

En particulier, on peut prévoir des implants :
- dont les corps 10 présentent des hauteurs différentes, avec dans ce cas des organes de renforcement d'ancrage 20 dont les diamètres peuvent évoluer pour s'adapter à ces différentes hauteur ;
- dont les corps présentent des largeurs différentes ; ainsi l'on peut prévoir, dans le cas particulier de la troisième forme de réalisation, une gamme d'implants dont les largeurs varient progressivement entre une largeur minimale (cas des figures 16 à 19) et une largeur maximale (par exemple telle que représentée sur les figures 33 à 44), en faisant varier l'épaisseur en direction latérale des branches latérales 102 et 103 du corps, tout en conservant la même taille d'espace intérieur et donc tout en pouvant utiliser le même organe 20 dans tous les cas avantageusement, ces branches latérales 102, 103 passent d'une simple paroi (figures 16 à 19) à une double paroi (figures 33 à 44) dès que l'épaisseur desdites branches 102, 103 est devenue suffisante pour permettre de pratiquer les ouvertures traversantes verticales 110 et 111;
- dont les corps présentent des faces supérieures et inférieures d'inclinaisons mutuelles différentes, aussi bien de l'avant vers Carrière que latéralement, avec des organes 20 de diamètres identiques ou différents ;
- dont les corps 10 et/ou les organes de renforcement d'ancrage 20 présentent des longueurs différentes ;
- dont les organes de renforcement d'ancrage présentent des saillies d'ancrage différentes, et en particulier plus ou moins profondes et plus ou moins espacées, selon les caractéristiques mécaniques rencontrées au niveau des plateaux vertébraux ; etc.

Bien entendu, la présente invention n'est nullement limitée aux formes de réalisation décrites ci-dessus et illustrées sur les dessins, mais l'homme du métier saura y apporter toute variante ou modification conforme à son esprit, et en particulier combiner entre elles les particularités des diverses formes de réalisation décrites.

Par ailleurs, les saillies d'ancrage osseux telles que décrites plus haut peuvent être constituées par tout moyen permettant d'assurer un ancrage mécanique et/ou par liaison osseuse avec les plateaux vertébraux sus- et sous-jacents. En particulier, il peut s'agir de revêtement poreux ou d'hydroxyapatite.

### DEMANDE DE BASE

Au dépôt, la demande de base dont est issue cette demande revendiquait un implant pour la chirurgie du rachis, comprenant un corps essentiellement creux apte à être Inséré dans un espace intervertébral, ledit corps possédant une paire de parois latérales entourant un espace intérieur exposé aux vertébres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit corps creux possédant des surfaces supérieure et inférieure pourvues de dents à bords vifs aptes à être ancrées dans lesdites vertèbres afin de s'opposer au mouvement inverse dudit corps hors dudit espace intervertébral.

Un aspect préféré mais non limitatif de cet implant est que les dents présentent une section transversale triangulaire

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel lesdites parois latérales du corps creux sont partiellement cylindriques et coaxiales avec un axe dudit organe de renforcement d'ancrage.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à entraîné en rotation dans l'espace intérieur du corps, et dans lequel lesdites parois latérales possèdent des ouvertures traversantes autorisant une croissance osseuse à travers elles.

Des aspects préférés mais non limitatif de cet implant sont que :
- lesdites ouvertures comprennent des fentes allongées s'étendant essentiellement parallèlement à la direction d'insertion dudit organe dans ledit corps creux,
- les parois latérales du corps sont des parois épaisses dans lesquelles sont formées des secondes ouvertures traversantes s'étendant entre les supérieure et inférieure du corps,
- lesdites premières ouvertures traversantes mettent en communication ledit espace intérieur avec lesdites secondes ouvertures traversantes,
- il est prévu des ouvertures traversantes mettant en communication lesdites secondes ouvertures traversantes avec les côtés extérieurs du corps.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans intérieur du corps, et dans lequel ledit corps creux possède une paroi d'extrémité distale reliant lesdites parois latérales, ladite paroi d'extrémité distale étant arrondie pour faciliter l'insertion dudit corps creux dans ledit espace intervertébral.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancra osseux inscrites dans un diamètre supérieur à la hauteur hors-tout du corps, ledit organe étant apte à être entraîné en rotation dans l'espace intérieur du corps, et dans lequel ledit corps creux possède une paroi d'extrémité distale reliant lesdites parois latérales, ladite paroi d'extrémité possédant un trou taraudé pour la fixation temporaire dudit corps creux à un instrument d'insertion du corps.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel lesdites saillies d'ancrage osseux comprennent un filetage de vis autotaraudeur,

Des aspects préférés mais non limitatif de cet implant sont que :
- ledit filetage de vis présente une section radiale généralement quadrangulaire,
- ledit filetage de vis présente une section radiale qui passe progressivement d'une section radiale essentiellement triangulaire à ladite section radiale genéralement quadrangulaire à partir de l'extrémité distale dudit filetage,
- le diamètre du filetage de vis augmente progressivement à partir de son extrémité distale jusqu'à une partie de diamètre essentiellement constant.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel lesdites saillies d'ancrage osseux comprennent un filetage de vis sous la forme d'une bande en hélice encerclant un espace intérieur dudit organe de renforcement d'ancrage.

Des aspects préférés non limitatif de cet implant sont que :
- ladite bande en hélice est reliée à une fourche s'étendant à l'intérieur de ladite bande dans une direction axiale dudit organe,
- ladite fourche comprend deux branches s'étendant à partir d'une paroi d'extrémité proximale dudit organe de renforcement d'ancrage,
- ladite fourche comprend des branches présentant une surface extérieure en forme de partie de tronc de cône dont le diamètre diminue de l'extrémité proximale vers l'extrémité distale de l'organe,
- ladite fourche comprend au moins deux branches possédant chacune un bord tranchant d'attaque de l'os,
- la fourche et la bande en hélice sont réalisées d'un seul tenant.

La demande de base telle que déposée revendiquait également un implant pour la chirurgie du rachis, comprenant un corps essentiellement creux apte à être inséré dans un espace intervertébral, ledit corps possédant une paire de parois latérales entourant un espacé intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, dans lequel lesdites saillies d'ancrage osseux comprennent un filetage de vis, et dans lequel ledit organe possède des moyens de blocage de celui-ci contre une rotation inverse.

Un aspect préféré mais non limitatif de cet implant est que lesdites saillies d'ancrage osseux comprennent un filetage de vis et lesdits moyens de blocage comprennent une partie déformée dudit filetage dans la région de son extrémité proximale.

La demande de base telle que déposée revendiquait également un implant pour la chirurgie du rachis, comprenant un corps essentiellement creux apte à être inséré ans un espace intervertébral, ledit corps possédant une paire de parois latérales entourant un espace intérieur exposé aux vertèbres sus- et sous-jacente qui définissent ledit espace intervertébral ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel lesdites saillies d'ancrage osseux comprennent un filetage de vis possédant un diamètre extérieur qui diminue dans sa région distale vers son extrémité distale.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit organe de renforcement d'ancrage possède une paroi d'extrémité proximale apte à essentiellement fermer une ouverture frontale dudit corps creux, de telle sorte qu'une substance promotrice de croissance osseuse placée à l'intérieur dudit organe soit comprimée pendant l'insertion dudit organe dans corps creux.

Des aspects préférés mais non limitatif de cet implant sont que :
- ledit organe de renforcement d'ancrage possède au moins une partie dont la surface externe appartient à un tronc de cône,
- ledit organe de renforcement d'ancrage est sensiblement plus court que le corps et possède une pointe généralement conique dirigée vers ladite paroi d'extrémité distale du corps,
- ladite paroi d'extrémité proximale possède une ouverture taraudée pour la fixation temporaire dudit organe à un instrument d'insertion de l'organe.

La demande de base telle que déposée revendiquait également un implant pour la chirurgie du rachis, comprenant un corps essentiellement creux apte à être inséré dans un espace Intervertébral, ledit corps, possédant une paire de parois latérales entourant un espace intérieur exposé aux vertèbres sus- et sous-jacente qui définissent ledit espace Intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit organe de renforcement d'ancrage possède des moyens d'indexation pour fixer ledit organe à un instrument d'insertion de l'organe dans une relation angulaire donnée.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage creux possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit organe de renforcement d'ancrage possède à son extrémité proximale un bouchon rapporté.

Des aspects préférés mais non limitatif de cet implant sont que :
- ledit bouchon est vissé dans une ouverture frontale taraudée de l'organe de renforcement d'ancrage,
- ledit bouchon est engagé par encliquetage élastique dans une ouverture frontale de l'organe de renforcement d'ancrage,
- ledit bouchon possède un aménagement apte à coopérer avec un instrument permettant d'entraîner l'organe en rotation,
- ledit bouchon possède des aménagement d'indexation angulaire de l'organe de renforcement d'ancrage avec un instrument de pose dudit organe.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel lesdites saillies d'ancrage osseux comprennent un filetage de vis, et au moins l'une des branches latérales du corps possède une partie rentrante formant filet apte à coopérer avec ledit filetage.

Des aspects préférés mais non limitatif de cet implant sont que :
- ladite partie rentrante est prévue seulement sur l'une des branches et constitue la seule partie du corps coopérant par vissage avec le filetage de vis,
- ladite partie rentrante présente un bord d'extrémité libre essentiellement rectiligne.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit organe est orienté en oblique par rapport à un plan du corps correspondant au plan sagittal.

Un aspect préféré mais non limitatif de cet implant est que l'organe de renforcement d'ancrage est orienté à approximativement 45° par rapport à un plan du corps correspondant au plan sagittal.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage creux possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel des ouvertures traversantes sont prévues ans ledit organe entre l'intérieur et l'extérieur de celui-ci, lesdites ouvertures étant allongées dans une direction essentiellement circonférentielle de l'organe.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit corps possède une paroi d'extrémité distale, et ledit organe de renforcement d'ancrage possède une partie d'extrémité distale apte à être vissée dans une ouverture de ladite paroi d'extrémité distale.

Un aspect préféré mais non limitatif de cet implant est que ledit corps possède également une paroi d'extrémité proximale incluant une ouverture plus large que la dimension extérieure dudit organe de renforcement d'ancrage et dans laquelle le dit organe peut être engagé librement.

La demande de base telle que déposée revendiquait également un implant pour la chirurgie du rachis, comprenant un corps essentiellement creux apte à être inséré dans un espace intervertébral, ledit corps possédant une paire de parois latérales entourant un espacé intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel le corps présente une paroi proximale, une paroi distale et deux parois latérales, lesdites parois définissant entre elles un espace intérieur plus grand que ledit organe de renforcement d'ancrage.

Des aspects préférés mais non limitatif de cet implant sont que :
- ledit organe de renforcement d'ancrage possède une partie filetée pour son vissage dans la paroi proximale du corps,
- ledit organe de renforcement d'ancrage et la paroi distale du corps possèdent des moyens filetés coopérant mutuellement pour la fixation dudit organe au corps,
- lesdites saillies d'ancrage osseux comprennent un filetage de vis ayant le même pas que ladite partie filetée de fixation au corps,
- les parois latérales et la paroi proximale du corps s'étendent essentiellement sur un même arc de cercle, et ladite paroi distale est essentiellement rectiligne,
- les faces supérieure et inférieure du corps possèdent des saillies d'ancrage osseux s'étendant le long de ses parois.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel des moyens sont prévus pour le montage à rotation de l'organe de renforcement d'ancrage dans l'espace intérieur du corps tout en empêchant un mouvement de translation relative entre ceux-ci.

Des aspects préférés mais non limitatif de cet implant sont que :
- les moyens de montage comprennent une ouverture cylindrique formée dans une paroi d'extrémité distale dudit corps et un arbre prévu sur ledit organe et apte à être engagé par déformation élastique dans ladite ouverture,
- ledit organe de renforcement d'ancrage présente la forme d'une vis possédant deux plats diamétralement opposés, lesdites saillies d'ancrage osseux étant définies entre lesdits plats et des bords tranchants étant prévus aux transitions entre le filetage de la vis et les plats,
- la distance entre les plats opposés n'est pas supérieure à la distance entre les faces supérieure et inférieure dudit corps.

La demande de base telle que déposée revendiquait également un implant pour la chirurgie du rachis, comprenant un corps essentiellement creux apte à être inséré dans un espace intervertébral, ledit corps possédant un ensemble de parois généralement parallèles définissant au moins deux espaces intérieurs situés côte-à-côte et exposés aux vertèbres sus- et sous-jacente qui définissent ledit espace intervertébral, ledit implant comprenant en outre au moins deux organes de renforcement d'ancrage possédant sur leur surface extérieure des saillies d'ancrage osseux inscrites dans un diamètre supérieur à la hauteur hors-tout du corps, lesdits organes étant aptes à être entraînés en rotation dans des espaces intérieurs respectifs dudit corps.

Un aspect préféré mais non limitatif de cet implant est que lesdits organes sont identiques,

La demande de base telle que dépasée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit corps creux des surfaces supérieure et inférieure qui sont inclinées l'une par rapport à l'autre, avec une distance entre elles qui diminue de l'extrémité proximale vers l'extrémité distale du corps.

La demande de base telle que déposée revendiquait également un implant pour la chirurgie du rachis, comprenant un corps essentiellement creux apte à être inséré dans un espace intervertébral, ledit corps possédant une paire de parois latérales entourant un espace intérieur exposé aux vertèbres sus- et sous-jacente qui définissent ledit espace intervertébral, ledit implant comprenant en outre un organe de renforcement d'ancrage possédant sur sa surface extérieure des saillies d'ancrage osseux inscrites dans un diamètre supérieur à la hauteur hors-tout du corps, ledit organe étant apte à être entraîné en rotation dans l'espace intérieur du corps, et dans lequel ledit corps creux possède des surfaces supérieure et inférieure qui sont inclinées l'une par rapport à l'autre, avec une distance entre elles qui diminue d'un premier côté latéral du corps vers le côté latéral opposé.

La demande de base telle que déposée revendiquait également un jeu d'implants pour former un implant rachidien destiné à être inséré dans un espace intervertébral de la colonne vertébrale humaine en étant adapté à la géométrie dudit espace intervertébral, ledit jeu comprenant :
une pluralité de corps creux possédant chacun une paire de parois latérales délimitant un espace intérieur et aptes chacun à être inséré dans un espace intervertébral de telle manière que ledit espace intérieur soit exposé aux vertèbres sus- et sous-jacente qui définissent ledit espace intervertrébral chacun desdits corps ayant une taille et une forme spécifiques,
au moins un organe de renforcement d'ancrage possédant sur sa surface extérieure des saillies d'ancrage osseux inscrites dans un diamètre supérieur à la hauteur hors-tout des corps, ledit organe étant apte à être entraîné en rotation dans l'espace intérieur de l'un quelconque desdits corps,
de telle sorte qu'un corps creux spécifique convenant à la configuration particulière d'un espace intervertébral donné puisse être choisi parmi ladite pluralité de corps creux.

Des aspects préférés mais non limitatif de ce jeu d'implants sont que
- ladite pluralité de corps creux inclut un groupe de corps creux possédant différents angles d'inclinaison entre leurs surfaces supérieures et inférieures,
- ladite pluralité de corps creux inclut un groupe de corps creux différentes largeurs,
- les corps creux les plus larges présentent des parois latérales dans lesquelles sont formées des ouvertures traversantes s'étendant entre les faces supérieures et inférieures desdits corps,
- ladite pluralité de corps creux inclut un groupe de corps creux présentant différentes hauteurs,
- laite pluralité de corps creux inclut un groupe de corps creux présentant différentes longueurs,
- un groupe déterminé de corps creux de ladite pluralité est apte à recevoir un même type d'organe de renforcement d'ancrage.

La demande de base telle que déposée revendiquait également un procédé pour positionner dans un espace intervertébral d'une colonne vertébrale humaine un implant comprenant un corps essentiellement creux possédant une paire de parois latérales entourant un espace intérieur et un organe de renforcement d'ancrage possédant sur sa surface extérieure des saillies d'ancrage osseux inscrites dans un diamètre supérieur à la hauteur hors-tout du corps et apte à être entraîné en rotation dans l'espace intérieur du corps, ledit procédé comprenant les étapes suivantes :
sélectionner à partir d'un jeu de corps creux possédant différentes formes et dimensions un corps creux adapté à la configuration dudit espace intervertébral
remplir ledit corps creux sélectionné avec une substance promotrice de croissance osseuse
pousser ledit corps creux dans ledit espace intervertébral de telle manière que l'espace intérieur de ceiui-ci soit exposé aux vertèbres sus- et sous-jacente qui définissent ledit espace intervertébral ; et
insérer ledit organe de renforcement d'ancrage dans ledit corps creux de manière que lesdites saillies d'ancrage osseux s'ancrent dans lesdites vertèbres sus et sous-jacente.

Un aspect préféré mais non limitatif du procédé ci-dessus est que, pour le positionnement d'un implant dont le corps présente des parois latérales pourvues d'ouvertures traversantes s'étendant entre les faces supérieure et inférieure dudit corps, comprenant en outre, avant l'étape de poussée dudit corps creux dans ledit espacé intervertébral, une étape de remplissage ouvertures traversantes avec une -substance promotrice de croissance osseuse.

## Revendications

1. Un implant pour la chirurgie du comprenant:
un corps (10) comportant des première et deuxième extrémités, deux parois latérales (102, 103) et des surfaces supérieure (104) et inférieure (104'),
**caractérisé en ce que** les surfaces supérieure (104) et inférieure (104') présentent l'une par rapport à l'autre une double obliquité, d'une part de l'une des parois latérales vers l'autre des parois latérales du corps, et d'autre part de la première extrémité vers la deuxième extrémité du corps.

2. Implant selon la revendication 1, **caractérisé en ce qu'**il comporte en outre un organe d'ancrage (20) destiné à être inséré dans le corps (10).

3. Implant selon la revendication 1, **caractérisé en ce que** les parois latérales présentent en coupe une forme concave et des surfaces internes concaves.

4. Implant selon la revendication 3, **caractérisé en ce que** les surfaces internes concaves comportent des portions non filetées définissant un espace interne au moins partiellement cylindrique entre les parois latérales.

5. Implant selon la revendication 1, **caractérisé en ce que** la distance entre les surfaces supérieure et inférieure diminue de l'une des parois latérales du corps vers l'autre des parois latérales, et de la première extrémité vers la deuxième extrémité du corps.

6. Implant selon la revendication 2, **caractérisé en ce que** l'organe d'ancrage (20) comporte des saillies d'ancrage (22).

7. Implant selon la revendication 6, **caractérisé en ce que** les saillies d'ancrages comprennent un filetage de vis sur une surface extérieure de l'organe d'ancrage.

8. Implant selon la revendication 7, **caractérisé en ce qu'**au moins l'une des parois latérales comporte un filet rentrant (108) destiné à coopérer avec le filetage de vis de l'organe d'ancrage.

9. Implant selon la revendication 8, **caractérisé en ce que** le filet rentrant (108 comporte un bord (108a) généralement rectiligne.

10. Implant selon la revendication 2, **caractérisé en ce que** l'organe d'ancrage (20) présente un diamètre extérieur supérieur à une hauteur du corps (10).

11. Implant selon la revendication 1, **caractérisé en ce qu'**au moins une des parois latérales comprend une première ouverture (106,106' ; 107, 107') pour permettre une repousse osseuse à travers la au moins une des parois latérales.

12. Implant selon la revendication 11, **caractérisé en ce que** la au moins une des parois latérales comporte une deuxième ouverture (110,111) s'étendant de la surface supérieure (104) à la surface inférieure (104').

13. Implant selon la revendication 12, **caractérisé en ce que** la deuxième ouverture est en communication avec la première ouverture.
